# EUROPEAN PATENT APPLICATION

(11) **EP 3 015 860 A1**
(43) Date of publication of application: **04.05.2016**
(21) Application number: 14804005.8
(22) Date of filing: 30.05.2014
(51) Int. Cl.: G01N 33/53, G01N 33/68, C07K 1/00, C12N 15/09

(54) **SUPER-COMPLEX FORMED BY CROSS-BINDING BETWEEN COMPLEXES OF REPEATING CHAIN AND MONOMER, AND USE THEREOF**

(30) Priority: 30.05.2013 KR 20130061869
(71) Applicant: Choe, Muhyeon, Seoul 137-882 (KR)
(72) Inventor: Choe, Muhyeon, Seoul 137-882 (KR)
(74) Representative: Brady, Paul Andrew
(86) International application number: PCT/KR2014/004862
(87) International publication number: WO 2014/193193

(57) **Abstract**

The present invention relates to a super-complex formed by a cross-binding between complexes of a repeating chain and a monomer, and a method for amplifying the effect of a monomer by forming the super-complex, and specifically, to: a super-complex generated by forming complexes of a repeating chain and a monomer, which contains the repeating chain of a binding domain having a binding specificity to the monomer as an active ingredient, and a cross-binding between the complexes; and a method for amplifying the effect of a monomer including signal amplification and the like by providing effects many times greater than that of the biological and chemical effects of a monomer for a monomer target since a plurality of monomers are contained in the super-complex.

## Description

### BACKGROUND OF THE INVENTION

### 1. Field of the Invention

The present invention relates to a super-complex formed by a cross-binding between complexes of a repeating chain and a monomer, and a method for amplifying the biochemical effect of a monomer by the same.

Particularly, a repeating chain/monomer complex is formed by comprising a repeating chain of a binding domain having a binding specificity to a monomer as an active component, from which a super-complex is formed by a cross-binding between the said complexes as the form of an aggregate. This super-complex comprises multiple numbers of monomers, so that it can provide the amplified monomer effect, specifically many times greater biological and chemical effect on a monomer target than a single monomer.

Herein the repeating chain can be prepared with some domains or materials that display a binding specificity to a natural monomer. The monomers to which the said binder could bind are in diversity. Ligands, receptors, antibodies, and enzymes are the examples. The effect of a monomer can be increased significantly by using multiple numbers of complexes prepared with monomers and repeating chains and a super-complex prepared with the complexes. In this monomer, biological and chemical functional groups are found as linked, conjugated, and fused, and at this time the effect of such functional groups can be amplified significantly.

### 2. Description of the Related Art

To detect a target, a monomer binds to the target and generates a detection signal. At this time, the intensity of the detection signal, the effect of the monomer, is determined by the binding affinity between the target concentration detection material and the detection probe, the probe concentration, and the strength of the probe signal. In general, the probe is a monomer, and the detection target material is a target material of the monomer, and the signal indicates the effect of the monomer.

Immunochromatographic assay, one of the detection methods using antibody, is also called 'rapid antigen test', 'lateral flow test', or simply 'strip test', which is widely applied for the development of a diagnostic kit. The target of diagnosis with the said immunochromatographic assay, reported so far, includes drug abuse, blood components analysis, group A streptococcal antigen, Helicobacter pylori, human Mycobacterium tuberculosis, hepatitis B surface antigen/antibody, Dengue virus, influenza, parasite (plasmodium falciparum for the diagnosis of malaria), etc. Immunochromatographic assay is simple and the duration of the assay is only about 5 ~ 10 minutes, which is very quick. The preservation at room temperature is excellent and the test cost is not expensive. So, this can be considered as the optimum method for the diagnosis of a disease that can be applied to various tests.

Colloidal gold is widely used as a chromogen for the immunochromatographic assay, and the colloidal gold-labeled immunoglobulin is widely used for the diagnosis of disease. The immunoglobulin conjugated colloidal gold particles have been applied for the direct detection of antigen molecules. In 1981, Leuvering et al developed gold particle agglutination assay so called sol particle immunoassay (SPIA), and further developed a pregnancy diagnostic kit using thereof. Since then, membrane test methods using gold have been reported for the diagnosis of diseases caused by bacteria, viruses, parasites, and fungi, etc. Rapid antigen immunoassay has also been developed as a quick field diagnosis method using colloidal gold which is easy and simple and the duration of the test is also as short as 5 ~ 10 minutes. Rapid antigen test is important since it is not only useful for the early diagnosis of human infectious diseases but also useful for the early diagnosis and prevention of animal infectious diseases as well.

The problem of the rapid antigen test kit used these days is that it is only effective within 2 ~ 3 days from the first acute disease symptom. For example, in the case of diagnosis of a virus caused disease, if the test is performed 3 days after the first symptom, the rapid antigen test kit may show negative result because the virus disappears rapidly 3 days after causing the symptoms. If a patient is a kid, the maintenance of the virus concentration is longer, so that the test sample still can be picked up after 5 days from the symptom development. However, if a patient is an adult, the virus concentration drops rapidly as time goes by, so the test sample has to be picked up within 4 ~ 5 days from the symptom development, suggesting that the antigen diagnosis is limited according to ages. Another problem of the conventional rapid antigen test kit is that the sensitivity of the diagnostic kit reagent is lower than expected, particularly to the low concentration of the antigen concentration. Various rapid antigen test reagents for the diagnosis of various influenza viruses have been developed world-widely and the sensitivity of these products increases continuously. However, the sensitivity to seasonal diseases is only 60 ~ 83% and the sensitivity to the novel swine virus is only 40 ~ 69%. The effect of three diagnostic tests on the market in USA nowadays, 'BinaxNow', 'EZ flu A+B (Becton, Dickinson and Company)', and 'Quickvue (Quidel)' was examined and as a result, the H1N1 virus detection efficiency of BinaxNow was 40%, which was the lowest, and Quickvue and EZ-flu A+B displayed respectively the efficiency of 69% and 49% (Centers for Disease Control and Prevention (CDC), Evaluation of rapid influenza diagnostic tests for detection of novel influenza A (H1N1) virus: United States, 2009. Morb Mortal Wkly Rep 2009; 58:826-829).

The reason of such a low sensitivity of the conventional rapid antigen test reagent is that the virus concentration was not enough because either the virus concentration in an infectee was very low or the sample was not picked up from a patient under the best/optimum condition to guarantee enough viruses for the test. Therefore, if a sample from a patient does not contain enough amount of antigen even though the patient has been diagnosed as infected with virus, it is very difficult to diagnose a disease. So, the study on the method and material useful for the detection of an antigen at a low concentration in a sample is required.

Previously, the present inventors constructed a complex comprising a monomer and a repeating chain by using a repeating chain of the binder having a binding specificity to the antigen Fab against such a monomer as antigen-toxin as the matrix (scaffold); succeeded in increasing the collision frequency of monomers by increasing the local concentrations of monomers; and accordingly developed a method to increase the yield of a cross-linked multimer by promoting the formation of a cross-binding (Korean Patent No. 10-1161323). The present invention provides a method to mass-produce a cross-linked multimer by promoting the formation of a cross-binding between monomers by using the repeating chain of a binder having a binding specificity to the monomers. However, the method to amplify the effect of an antibody by applying such a binding domain repeating chain having a binding specificity to the monomer to immunoassay has not been reported yet.

The study of the present inventors was focused on the promotion of the detection sensitivity in the course of diagnosis of a low concentration antigen. As a result, the inventors confirmed that the sensitivity to a low concentration antigen could be increased when the repeating chain of a binding domain having a binding specificity to an antibody monomer was used as a signal amplifier for such antigen detection methods as Western blotting, enzyme-linked immunosorbent assay (ELISA), and FACS (fluorescence activated cell sorter). The inventors accordingly confirmed that an antibody could be efficiently used as a detection monomer to amplify the effect of the detection antibody monomer in an antigen detection assay. There is no limit in the pairs of a monomer applicable to the present invention and a corresponding binding domain.

### SUMMARY OF THE INVENTION

It is an object of the present invention to provide a super-complex formed by a cross-binding between complexes of a repeating chain and a monomer, precisely a super-complex generated by forming complexes of a repeating chain and a monomer, which contains the repeating chain of a binding domain having a binding specificity to the monomer as an active ingredient, and a cross-binding between the complexes.

It is another object of the present invention to provide a method for amplifying the effect of a monomer by using the super-complex, wherein the super-complex contains multiple numbers of monomers so that it has many times higher biological/chemical effect on the monomer targets than a single monomer can have, resulting in the amplification of the monomer effect.

To achieve the above objects, the present invention provides the following [1] ~ [13].
[1] The present invention provides a method for preparing a repeating chain for the production of a super-complex containing the step of producing a repeating chain which comprises a single type of binding domains or multiple types of binding domains having at least two binding sites in a monomer and a binding specificity to the monomer.
[2] The present invention provides a method for preparing a complex of multiple numbers of monomers/repeating chains for the production of a super-complex, which comprises the following steps: 1) preparing a repeating chain wherein a single type of binding domains or multiple types of binding domains having at least two binding sites in a monomer and a binding specificity to the monomer are repeated; and 2) preparing a complex of multiple numbers of monomers/repeating chains by mixing the repeating chain of step 1) and the monomer having at least two binding sites binding to the said repeating chain.
[3] The present invention provides a method for preparing a super-complex, which comprises the following steps: 1) preparing a repeating chain wherein a single type of binding domains or multiple types of binding domains having at least two binding sites in a monomer and a binding specificity to the monomer are repeated; 2) preparing a complex of multiple numbers of monomers/repeating chains by mixing the repeating chain of step 1) and the monomer having at least two binding sites binding to the said repeating chain; and 3) forming an aggregate of the said complexes by forming a cross-binding between the complexes of multiple numbers of monomers/repeating chains of step 2).
[4] The present invention provides the repeating chain prepared by the method of [1].
[5] The present invention provides the complex of multiple numbers of monomers/repeating chains prepared by the method of [2].
[6] The present invention provides the super-complex prepared by the method of [3].
[7] The present invention provides a method for amplifying the effect of a monomer by forming the super-complex binding to the monomer target by mixing the repeating chain of [4], the complex of multiple numbers of monomers/repeating chains of [5] or the super-complex of [6], and the monomer target.
[8] The present invention provides a kit for the analysis of biochemical functions, detection, diagnosis, and treatment comprising the monomer which is specific to a target of detection or to induce a specific biochemical function and contains two or more binding sites for the repeating chain in a monomer, and a repeating chain of a binding domain having a binding specificity to the monomer.
[9] The present invention provides a method for preparing a repeating chain-biochemical functional group which comprises the step of linking, conjugating, or fusing a biological/chemical effector group or a detection functional group to a repeating chain of a binding domain having a binding specificity to the monomer.
[10] The present invention provides a method for preparing a complex of multiple monomers/repeating chains-biological/chemical functional groups, which comprises the following steps: 1) preparing a repeating chain-biochemical functional group which comprises the step of linking, conjugating, or fusing a biological/chemical effector group or a detection functional group to a repeating chain of a binding domain having a binding specificity to the monomer; and 2) preparing a complex of multiple monomers/repeating chains-biological/chemical effector groups by mixing the repeating chain-biological/chemical effector group prepared in step 1) and the monomer.
[11] The present invention provides the repeating chain-biological/chemical effector group of a binding domain that binds specifically to the monomer, prepared by the method of [9].
[12] The present invention provides the complex of multiple monomers/repeating chains-biological/chemical effector groups prepared by the method of [10].
[13] The present invention provides a method for the analysis of biochemical functions, detection, diagnosis, and treatment of a target, which comprises the step of forming a super-complex wherein complex of multiple monomers/repeating chains-biological/chemical effector groups of [12] is mixed with the monomer target.

### ADVANTAGEOUS EFFECT

In this invention, multiple numbers of monomers bind to a repeating chain, leading to the formation of a complex. Then, an insoluble super-complex is formed by a cross-binding between the complexes, which is precipitated when it is included at a high concentration. The aggregation, precipitation, and the size of the super-complex depend on the monomer and the repeating chain structures. The repeating number of the binding domain in the repeating chain affects the cross-binding between complexes. In the meantime, the water-solubility and the molecular size of the monomer affect the chances of cross-binding between complexes. Since the said super-complex contains multiple numbers of monomers, it can provide many times higher biological and chemical effect on the monomer target than a single monomer, so that it is useful for the amplification of a detection signal, functional effect, and therapeutic effect by using an antibody, the representative monomer.

### BRIEF DESCRIPTION OF THE DRAWINGS

The application of the preferred embodiments of the present invention is best understood with reference to the accompanying drawings, wherein:
Figure 1 is a schematic diagram illustrating the construction of an expression plasmid including GR1 ~ 10. Figure 1a is a diagram illustrating the construction of pGR2 vector ~ pGR20 vector. Precisely, pGR1 vector was first constructed and pGR2 ~ pGR10 vectors were constructed by using the pGR1 vector and likewise pGR11 ~ pGR20 vectors were constructed. Each plasmid has one G4S linker between domain IIIs. Figure 1b illustrating that pGR2 series, such as pGR2-2, pGR2-3, and pGR2-4, contain respectively two, three, and four G4S linkers between two domain IIIs.
Figure 2 is a diagram illustrating the result of SDS-PAGE with the repeating chain of the purified protein G domain III. The purified repeating chain was analyzed by 16% SDS-PAGE. Lane 1 ~ lane 13 indicate GR1, GR2, GR2-2, GR2-3, GR2-4, GR3, GR4, GR5, GR6, GR7, GR8, GR9, and GR10 respectively.
Figure 3 is a diagram illustrating the result of size-exclusion chromatography of GR series complexes. Two vertical arrows indicate the peaks of the disulfide-dimer (left) [Fab-ext-PE38]2 and the monomer (right) Fab-ext-PE38. The right table presents the comparison of the molecular weights of GR complexes, wherein the apparent molecular weights of a monomer (■) and of disulfide-bridged dimer (•) are presented. The schematic diagram illustrates the complex of GR3 or GR7 and Fab-ext-PE38.

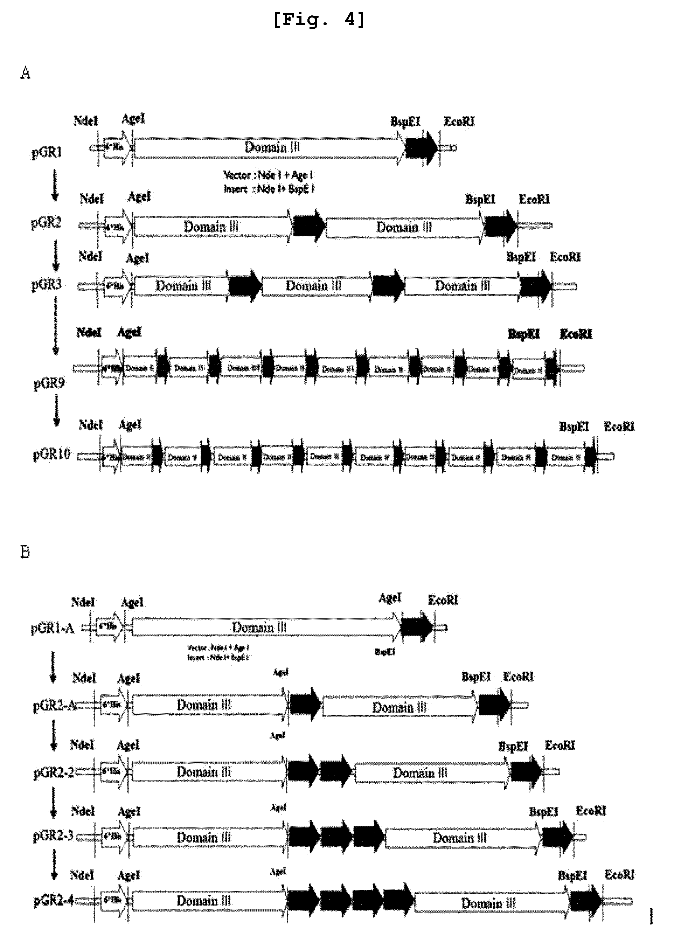

Figure 4 is a diagram illustrating the result of size-exclusion chromatography of GR2-2, GR2-3, and GR2-4 complexes. A indicates size-exclusion chromatography. One of the two vertical arrows, the left arrow indicates the peak of [Fab-ext-PE38]2 and the right arrow indicates the peak of Fab-ext-PE38. B illustrates the result of size-exclusion chromatography with the complexes of Fab-ext-PE38 and GR2-2, GR2-3, or GR2-4. The fractions were electrophoresed on non-reducing 8% polyacrylamide gel. The fractions corresponding to the elution volume of 9 ml ~ 15.5 ml were analyzed. The arrow indicates [Fab-ext-PE38]2. The arrowhead indicates Fab-ext-PE38. C indicates the electrophoresis performed on reducing 12% polyacrylamide gel. The arrow, the arrowhead, and the white open arrow indicate respectively Fd-ext-PE38, H6-L, and GR protein.

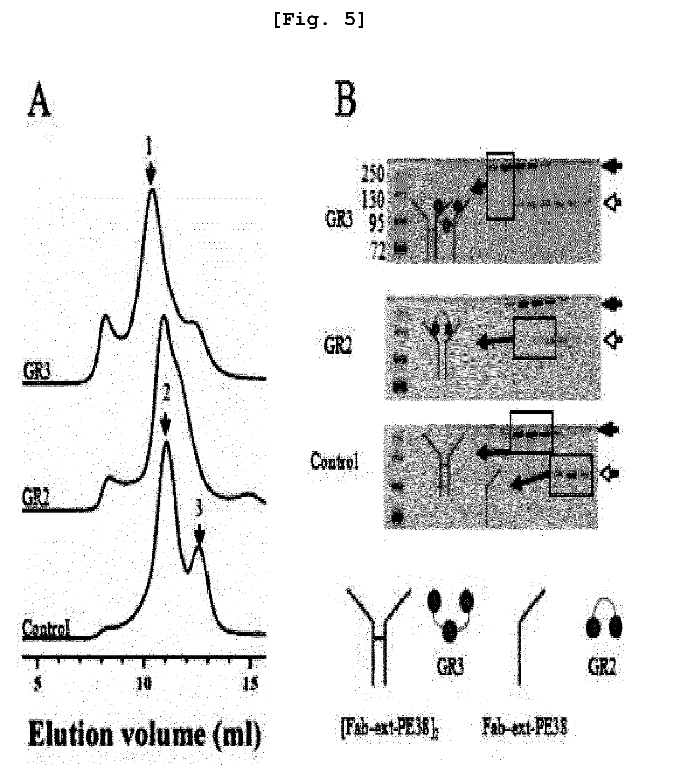

Figure 5 is a diagram presenting the result of the analysis of complexes of [Fab-ext-PE38]2 and Fab-ext-PE38 with GR2 or GR3. A indicates size-exclusion chromatography. 395 µg of the mixture of [Fab-ext-PE38]2 and Fab-ext-PE38 was mixed with GR2 or GR3 protein. At this time, 15 µg of GR protein was used. The vertical arrow and the number indicate the location of peaks. B presents SDS-PAGE with the eluted fraction. The mixture of [Fab-ext-PE38]2 and [Fab-ext-PE38] was used as the control, followed by electrophoresis on non-reducing 8% acrylamide gel. The fractions (#13 ~ #26) corresponding to the elution volume of 7 ml ~ 12.5 ml were compared. The black arrow and the white open arrow indicate respectively [Fab-ext-PE38]2 and Fab-ext-PE38.
Figure 6 is a diagram illustrating the result of size-exclusion chromatography with those proteins prepared by mixing Fab-PE38 monomer and GR2-2, GR2-3, or GR2-4. Those vertical arrows are the controls; the right arrow indicates Fab-PE38 monomer and the left arrow indicates disulfide-bridged dimer. The mixtures of Fab-PE38 monomers and GR2-2 ~ GR2-4 were over-lapped on chromatogram, suggesting that two Fab-PE38 monomers were included in the generated complex, which became a complex in the form of monomer dimer.

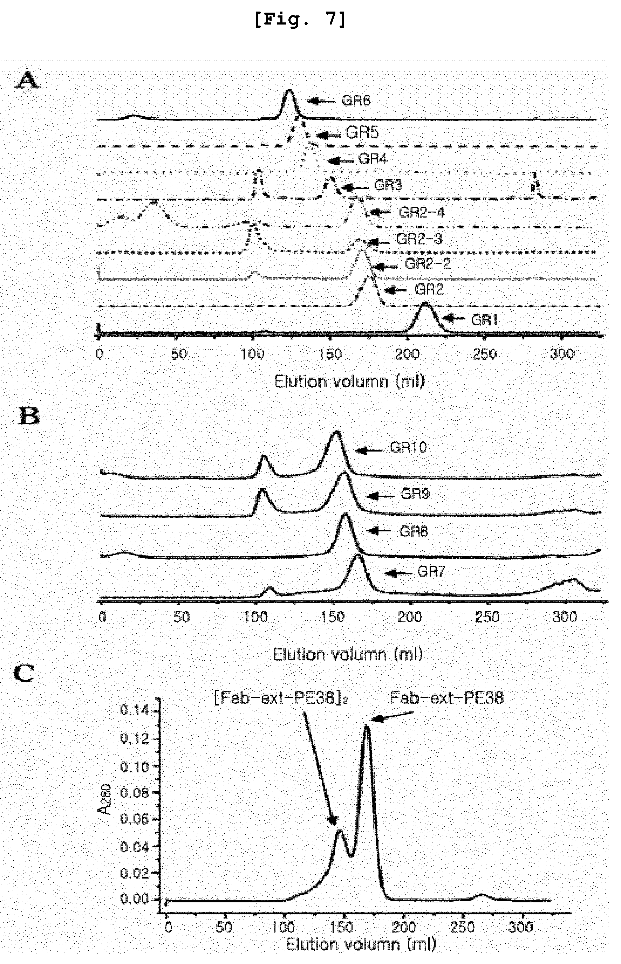

Figure 7 is a diagram illustrating the result of size-exclusion chromatography for the purification of the complex of GR repeating chain and Fab-PE38 protein. The protein was finally purified by size-exclusion chromatography. All the chromatograms are the records obtained at OD₂₈₀. The chromatograms were over-lapped according to the same elution volume. A: Hiload superdex-75 pg (26/60) column was used for the purification of GR1 ~ 6. B: Hiload superdex-200 pg (26/60) column was used for the purification of GR7 ~ 10. C: Hiload superdex-200 pg (26/60) column was used for the final purification of antibody-toxin.

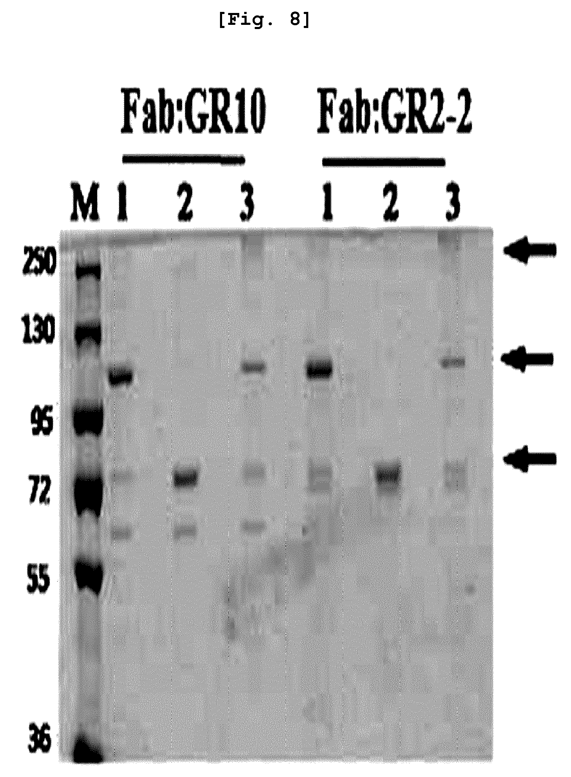

Figure 8 is a diagram illustrating the result of 8% non-reducing SDS-PAGE proving the formation of disulfide-bridged dimer from Fab-toxin monomer by oxidation-reduction reaction in the complex of Fab-toxin monomer and GR10 or GR2-2, GR2-3, or GR2-4. First pathway: Fab-toxin monomer, the reaction starting material. Second pathway: the sample reduced at room temperature with 40 mM 2-mercaptoethanol for 30 minutes. Third pathway: the sample oxidized with 5 mM glutathione oxidized form (GSSG) at 37 °C for 2 hours. Arrows indicate disulfide-bridged dimer, Fab-toxin monomer, and Fd chain, respectively from the top.
Figure 9 is a diagram illustrating the amplification of chemiluminescence signal by Western blotting with GR10 using the conventional Western blotting reagents; Lane A: 20 µg of A431 whole cell lysate (WCL) ; Lane 1: 2 µg of A431 WCL; Lane 2: 1 µg of A431 WCL; Lane 3: 0.5 µg of A431 WCL; GR10 treated Western blot: GR10 treated Western blotting provides the 32-fold amplified signal, compared with the conventional Western blotting.
Figure 10 is a diagram illustrating the amplification of Western blot chemiluminescence signal caused by GR10 repeating chain. a: 17 times higher signal provided by the super-complex prepared by the complex of the mouse anti-β-actin monoclonal antibody and GR10. b: the signal amplification by the super-complex which was similar to that of nitrocellulose membrane, wherein the super-complex was obtained after the separation of A431 clear cell lysate by 10% denaturing SDS-PAGE, followed by the transfer of the product onto PVDF membrane.

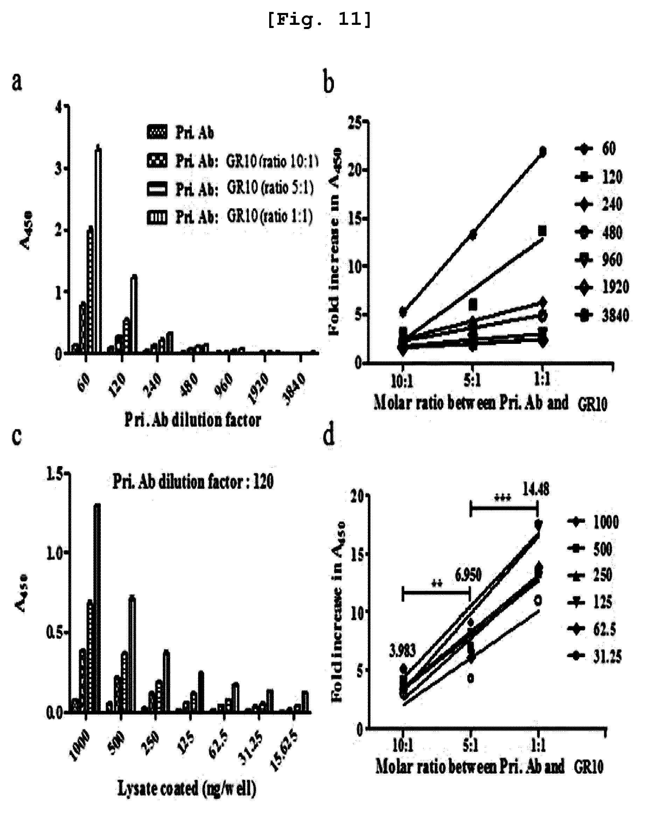

Figure 11 is a diagram illustrating the increase of ELISA sensitivity by GR10. a: Each well was coated with 1 g of AGS cell lysate. The primary antibody was serially diluted. b: A graph illustrating the increase of OD of A450, compared with when the primary antibody alone was treated, according to the different molar ratios of the primary antibody to GR10 which was added in order to form a super-complex, considering the dilution rate of the primary antibody. c: The primary antibody was fixed at the dilution rate of 1:120 and the AGS cell lysate was serially diluted. Then, each well of a 96-well plate was coated with the cell lysate. d: A graph illustrating the increase of OD of A450, compared with when the primary antibody alone was treated, according to the molar ratio of the primary antibody to GR10, which was added in order to form a super-complex with considering the concentration of the cell lysate coating each well.
Figure 12 is a diagram illustrating the effect of signal amplification according to the serial dilution of the secondary antibody.
Figure 13 is a diagram illustrating the increase of detection sensitivity of a rapid antigen test kit by GR10.
Figure 14 is a diagram illustrating the result of immunofluorescence with the human squamouse carcinoma cell line A431 by using the GR1-FITC conjugate.
Figure 15 is a diagram illustrating the cross-binding between the GR repeating chain and the test line antibody in the course of rapid antigen test.
Figure 16 is a diagram illustrating the cross-binding between the AR, LR, or LAR repeating chain and the test line antibody in the course of rapid antigen test.
Figure 17 is a diagram illustrating the increase of detection sensitivity of a rapid antigen test kit by GR5.
Figure 18 is a diagram illustrating the increase of detection sensitivity of a rapid antigen test kit by GR10.
Figure 19 is a diagram illustrating the increase of detection sensitivity of a rapid antigen test kit by GR15.
Figure 20 is a diagram illustrating the increase of detection sensitivity of a rapid antigen test kit by GR20.
Figure 21 is a diagram illustrating the increase of detection sensitivity of a rapid antigen test kit by AR5.
Figure 22 is a diagram illustrating the increase of detection sensitivity of a rapid antigen test kit by LR5.
Figure 23 is a diagram illustrating the increase of detection sensitivity of a rapid antigen test kit by LAR3.
Figure 24 is a diagram illustrating that GR10 formed a super-complex with IgG, and the super-complex was precipitated without being dissolved in an aqueous solution. IgG and GR10 were mixed at the molar ratio of 1:1, 5:1, and 10: 1, starting from the left, followed by reaction.

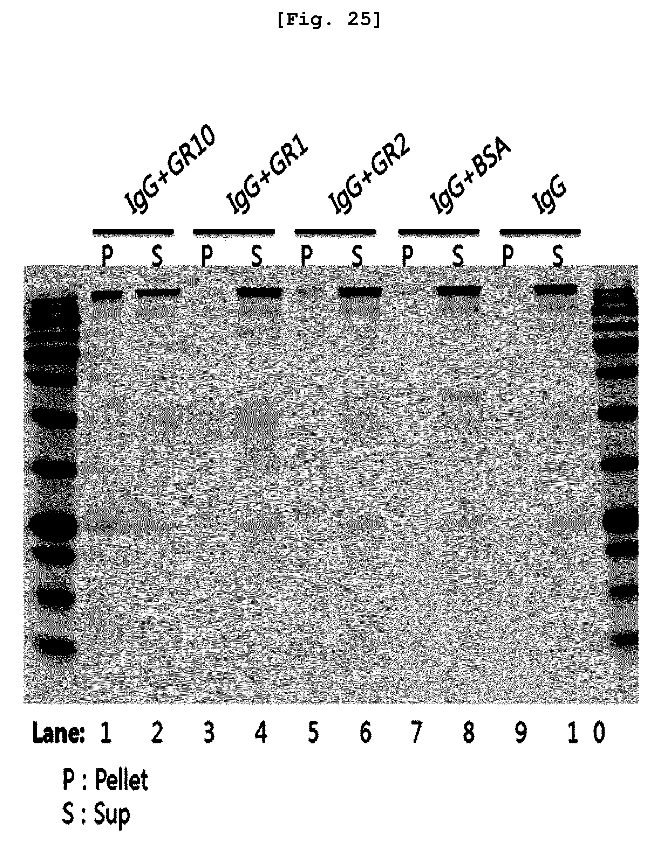

Figure 25 is a diagram illustrating the result of SDS-PAGE confirming that the GR/IgG super-complex precipitate was greater when IgG was mixed with GR10 than when IgG was mixed with GR1 or GR2. 1/36 of each sample was examined by non-reducing 15% SDS-polyacrylamide gel. Lane 1 and lane 2 are respectively the precipitates generated from the reaction of IgG and GR10 and the supernatant. Lane 3 ~ lane 6 are the precipitates generated from the reaction of IgG with GR1 and GR2 and the supernatants in that order. Lane 7 and lane 8 are the control samples prepared by the reaction of BSA and IgG, and lane 9 and lane 10 are the control samples containing IgG only.
Figure 26 is a diagram showing the precipitate generated by the formation of GR/IgG super-complex. GR1 ~ GR10 were reacted with IgG. As the controls, BSA was reacted with IgG and IgG alone was selected, followed by centrifugation at 13,000 rpm at 20°C for 30 minutes. The micro-centrifuge tube was turned upside down and the precipitate was confirmed by the naked eye. The circle indicates the formed precipitate.
Figure 27 is a diagram illustrating the result of SDS-PAGE that confirmed the precipitate generated by the formation of GR/IgG super-complex. Lane 1 ~ lane 20 indicate the precipitates generated by the reaction of GR1 ~ 10 with IgG and the supernatants in that order. Lane 21 and lane 22 are the controls which are the result of reaction between BSA and IgG. Lane 21 and lane 22 are the control samples prepared by the reaction of BSA and IgG, and lane 23 and lane 24 are the control samples containing IgG only. 1/30 of each sample was examined by reducing 15% SDS-polyacrylamide gel.
Figure 28 is a diagram illustating the result of SDS-PAGE to investigate whether or not GR greater than GR10 also could form a super-complex with IgG. Lane 1 ~ lane 12 are the precipitates generated from the reaction of GR1, 3, 5, 10, 15, and 20 with IgG and the suerpnatants in that order. Lane 13 and lane 14 are the control sample precipitate made of only IgG and the supernatant. 1/10 of each sample was examined by reducing 15% SDS-polyacrylamide gel.
Figure 29A is a diagram illustrating the comparison of the two Western blotting results, wherein the detection limit of Western blotting was extended by GR which was compared with the result of Western blotting without using GR. In Figure 29B, the result of Figure 29A was schemetized.
Figure 30A is a diagram illustrating the result of Western blotting confirming the amplification of chemiluminescence signal by GR in the presence of equal amount of antigen. Figure 30B is a graph illustrating the result of Figure 30A.
Figure 31A is a diagram illustrating that the amplification of Western blot signal by GR super-complex was confirmed using other antigens. Figure 31B is a diagram illustrating that the amplification of Western blot signal could be achieved by repeating chain in the presence of different primary antibody.
Figure 32A is a diagram illustrating the comparison of the amplification levels of Western blot signal by repeating chain. Precisely, this is a diagram illustrating the comparison of the amplification levels Western blot signal by GR10, AR10, and MAR5 (LAR5 repeating chain wherein L is different.) in the same condition. Figure 32B is a diagram showing the result of Western blotting using LR10.
Figure 33 is a diagram illustrating the cross-binding between the repeating chains of GR series proteins and the gold antibody complx or the super-complex detection line antibody.
Figure 34 is a diagram illustrating the cross-binding between the repeating chains of AR series proteins and the gold antibody complx or the super-complex detection line antibody.
Figure 35 is a diagram illustrating the cross-binding between the repeating chains of LR series proteins and the gold antibody complx or the super-complex detection line antibody.
Figure 36 is a diagram illustrating the cross-binding between the repeating chains of MAR series proteins and the gold antibody complx or the super-complex detection line antibody.
Figure 37 is a diagram illustrating the result of non-reducing SDS-PAGE by using the purified TR1, 3, 5, 10, 15, and 20 (=GR1, 3, 5, 10, 15, 20) proteins and B3(Fab)-ext-PE38 attached thereon that were reduced by 2-mercaptoethanol and then oxidized into glutathione oxidized form (GSSG) in order to produce [B3(Fab)-ext-PE38]2.
Figure 38 is a diagram illustrating the result of SDS-PAGE by using the purified GR5, GR10, GR15, and GR20 (=TR5, TR10, TR15, TR20) proteins and Herceptin(Fab)-ext-PE38 attached thereon that were reduced by 2-mercaptoethanol and then oxidized into glutathione oxidized form (GSSG) in order to produce [Herceptin(Fab)-ext-PE38]2.
Figure 39 is a diagram illustrating the cytotoxic effect of the complex of GR repeating chain protein and [Herceptin(Fab)-ext-PE38]2 monomer on SKBR3 cells and BT 474 cells.
Figure 40 is a diagram illustrating the cytotoxic effect of the complex of GR repeating chain protein and [e23(Fab)-ext-PE38] monomer on SKBR3 cells and BT 474 cells.

### DESCRIPTION OF THE PREFERRED EMBODIMENTS

Hereinafter, the terms used in this invention are defined.

The term "binder repeating chain" herein indicates the recombinant protein generated by the material wherein the site that has a binding specificity to a monomer such as an antibody is repeated. The site that has a binding specificity herein indicates a binding domain showing a binding specificity to a monomer. In a natural protein, the domain that binds specifically to a monomer is the site indicated.

The term "antibody monomer" used in this invention indicates the molecule originated from an antibody, which includes an antibody fragment or an antibody conjugated with other proteins or functional biological-chemical molecules, which is supposed to bind to the repeating chain. A natural antibody has two heavy chains and two light chains. When one heavy chain and one light chain make a pair in one unit, it is called a dimer, but in this invention, when a natural general antibody is conjugated with a repeating chain, it is called an "antibody monomer".

The term "multiple antibody monomers/repeating chain complex" in this invention indicates the complex prepared by contacting the said antibody monomer to the repeating chain of a binder that specifically binds to the said monomer.

The term "super-complex of multiple antibody monomers/repeating chain complex" indicates the super-complex that is the aggregate of complexes formed by a cross-binding between the complexes prepared by contacting the said antibody monomer to the repeating chain of a binder that binds specifically to the said monomer.

In addition, the term "antigen/multiple antibody monomers/repeating chain complex" used in this invention indicates the complex prepared by contacting the said multiple antibody monomers/repeating chain complex to an antigen.

The term "super-complex of antigen/multiple antibody monomers/repeating chain complex" in this invention indicates the conjugate of the super-complex and the antigen generated by binding between an antigen and the super-complex prepared by contacting the super-complex that is the aggregate of the complexes formed by a cross-binding between the multiple antibody monomers/repeating chain complexes to an antigen or by preparing the antigen/repeating chain mixture first and then adding this mixture to the antibody monomer in the presence of an antigen to form a super-complex.

Hereinafter, the present invention is described in detail.

The present invention provides a method for preparing a repeating chain containing the step of producing a repeating chain wherein a single type of binding domains or multiple types of binding domains which bind specifically to the monomer and contain at least two binding sites in one monomer are repeated.

The present invention also provides a method for preparing a complex of multiple monomers/repeating chain containing the step of preparing the multiple monomers/repeating chain complex by mixing the repeating chain prepared by the above method with multiple numbers of monomers.

The present invention also provides a method for preparing a super-complex containing the step of forming an aggregate of the said complexes by a cross-binding between the multiple monomers/repeating chain complexes prepared by the method above.

In the above method, the monomer is preferably a protein, which is more preferably selected from the group consisting of antibodies, ligands and receptors, fragments thereof, recombinant conjugates thereof, derivatives thereof, and biological/chemical effector group conjugates.

In the above method, the antibody is preferably selected from the group consisting of antibody fragments, Fab fragments, Fab fragment containing fragments, Fv fragments, Fv fragment containing fragments, Fc fragments, and Fc fragment containing fragments.

In the above method, the binding domain is preferably a protein, and more preferably a microorganism originated protein, which is more preferably selected from the group consisting of streptococcal protein G, Staphylococcus aureus protein A, Peptostreptococcus magnus protein L, and derivatives thereof.

In the repeating chain of the present invention, a flexible linker chain can be included in order for each domain to rotate freely in between the repeated binding domains therein and to maintain a distance between the domains. When monomers bind together, the collision between the monomers cause stereoscopic hindrance, resulting in the decrease of binding reaction rate constant, which suggests that the decrease of binding reaction equilibrium constant is prevented so that high binding reaction equilibrium can be obtained.

In the binding domain repeating chain, the binding domain can rotate freely and chain bending is also possible owing to the flexible linker in between the binding domains. Therefore, in the complex formed with a monomer and the repeating chain, each binding monomer can have rotational freedom and vibrational freedom along with bending freedom. So, each binding monomer can avoid collision between each other because it is allowed to move freely not just in a limited rotation direction or in a narrow bending angle but in a wide direction range and a wide angle range, so that multiple numbers of monomers can be bound to the repeating chain at the same time. The flexible linker sequence makes a big room between the binding domains, so that many monomers that are approaching to the repeating chain for the conjugation can bind to the repeating chain without any stereoscopic hindrance between each other. A natural protein having the binding domain that can be used for the repeating chain of the present invention does not have such flexibility between the binding domains. Natural proteins do not allow such degree of freedom to their binding domains inside, suggesting that a monomer that is approaching thereto in order to form a complex with multiple monomers or a monomer that has been bound already thereto cannot have such a high level of rotation freedom and vibration freedom.

The binding domain used for the repeating chain of the present invention is preferably a natural protein fragment, and this kind of natural protein fragment has a lower molecular weight than a whole natural protein. The repeating chain having more binding domains than a natural protein having a comparatively high molecular weight, from which the above fragment has been derived, and having a low molecular weight can be constructed with the artificially designed repeating chain prepared by using such low molecular weight binding domains. Accordingly, many monomers can bind to a low molecular weight repeating chain. The repeating chain that has many binding domains but has a low molecular weight is easy to produce and purify, which is a huge advantage that the natural protein cannot have. In this invention, a repeating chain wherein binding domain repeats 20 times has been constructed, but the number of repeating is not limited thereto.

When the repeating chain characterized by many binding domains but a low molecular weight is used, the effect of monomer can be amplified because the rate of the monomer to binding molecule (natural microorganism protein molecule, repeating chain in this invention) is high at this time, which cannot be obtained from a natural molecule. Since such a repeating chain has a low molecular weight, it is useful for the production of a synthetic protein.

In this invention, the monomer has to have the different sites a' and b' and the binder has to have the corresponding binding sites a and b, so that the repeating chain has to have at least one of the repeating a and b and the monomer has to have at least one of each a' and b' in order to form a super-complex comprising multiple monomers/repeating chain complexes.

In this invention, the monomer has to have a' and b' together in the form of (a'b'), and the binding domain in a repeating chain has to have the binding sites a and b together to form the repeats of (ab)-(ab)-(ab)----(ab) or the binding sites a and b stay independently in different binding domains that make the repeating in the form of a-b-a-b-a-b----a-b that means the independent binding sites a and b are repeated separately. The numbers and the order of the independent domains a and b in the repeating chain are not limited. When a=b, the monomer is (a'a') and the repeating chain is in the form of (aa)-(aa)-(aa)--- or a-a-a-a-a----- .

In this invention, the repeating chain for the formation of a complex by cross-binding can have the binding domains, c and d, that bind to each other, and at this time these domains are independent and not involved in the binding between the multiple monomers and the repeating chain. If the repeating chain is composed of c-a-a-a-------d, the said monomer binds to the domain a and the cross-binding between complexes is achieved by the binding between c and d. For example, a super-complex can be composed of c-a-a-a---a-d...c-a-a-a-----a-d....c-a-a-a-----a-d, wherein '....' indicates the cross-binding between the repeating chains. The super-complex formed thereafter can amplify the effect of the monomer bound to the repeating chain. At this time in the structure of the repeating chain, the domains c and d in one repeating chain need to be firm not to be bended so as not to bind to each other. If the domains c and d bind to each other in one repeating chain, the chances of the cross-binding between the multiple monomers/repeating chain complexes are very low, and accordingly the chances of the formation of a super-complex becomes very low. It is not desirable either that the domain c of one repeating chain binds to domain d of another repeating chain before the cross-binding with a monomer. If such a linking between two different repeating chains is made, the repeating chain is difficult to control and therefore it is hard to form a complex with monomers.

The present invention also provides a repeating chain wherein a kind of binding domain or different kinds of binding domains which bind specifically to the monomer and have at least two binding sites in one monomer are repeated.

The present invention also provides a complex of multiple monomers/repeating chain wherein multiple numbers of monomers are linked to the said repeating chain.

The present invention also provides a super-complex that is an aggregate of the complexes resulted from the cross-binding between the multiple monomers/repeating chain complexes.

The monomer herein is preferably a protein, which is more preferably selected from the group consisting of antibodies, ligands and receptors, fragments thereof, recombinant conjugates thereof, derivatives thereof, and biological/chemical effector group conjugates.

The antibody herein is preferably selected from the group consisting of antibody fragments, Fab fragments, Fab fragment containing fragments, Fv fragments, Fv fragment containing fragments, Fc fragments, and Fc fragment containing fragments.

The binding domain herein is preferably a protein, and more preferably a microorganism originated protein, which is more preferably selected from the group consisting of streptococcal protein G, Staphylococcus aureus protein A, Peptostreptococcus magnus protein L, and derivatives thereof.

The present invention also provides a method for amplifying the effect of a monomer containing the step of forming a super-complex on the target of the monomer by mixing the repeating chain of the present invention, the multiple monomers/repeating chain complexes of the invention, or the super-complexes thereof.

In the above method, the step of measuring the effect of the monomer on the target can be additionally included.

In the above method, the target of the monomer is preferably selected from the group consisting of antigens, antibodies, peptides, proteins, bacteria, viruses, and fungi, or their fragments, but not always limited thereto.

The said bacteria are preferably selected from the group consisting of Helicobacter pylori, Mycobacterium tuberculosis, and Chlamydia trachomatis, but not always limited thereto.

The said viruses are preferably selected from the group consisting of influenza, foot-and-mouth disease virus, human papilloma virus (HPV), dengue fever virus, hepatitis C virus, hepatitis B surface antigen, and hepatitis B surface antibody, but not always limited thereto.

In the above method, the measurement of the effect of the monomer is preferably performed by using a monomer-marker conjugate or a secondary detector(antibody)-marker conjugate, and a substrate of a marker, but not always limited thereto.

The formation of a super-complex in this invention is advantageous for the amplification of the effect of a monomer including signal amplification because the super-complex of the invention contains multiple numbers of monomers to increase the biological and chemical effect on the target of the monomer which is many times higher than a single monomer can provide.

The present invention provides an analysis kit comprising the repeating chain of a binding domain which is specific to a detection target and contains multiple numbers of monomers having at least two binding sites in one monomer and has a binding specificity to the monomer.

The kit is preferably composed of the followings:
1) repeating chain of binding domain having a binding specificity to monomer;
2) monomer specifically binding to the detection target;
3) secondary probe conjugate labeled with a marker activated by the reaction with a substrate;
4) substrate solution to react with the marker;
5) washing buffer for each reaction stage; and
6) stop solution to terminate the enzyme reaction, but not always limited thereto.

The kit herein can be used for the analysis method selected from the group consisting of immunohistochemical techniques, immunoblot, immunoprecipitation, enzyme linked immunosorbent assay (ELISA), agglutination, immunochromatographic assay, and radio-immuno assay.

The marker herein is preferably selected from the group consisting of horseradish peroxidase (HRP), alkaline phosphatase, colloid gold, fluorescein, Quantum dot, glucose oxidase, luciferase, beta-D-galactosidase, malate dehydrogenase (MDH), acetylcholinesterase, isotope, and dye, but not always limited thereto.

The substrate herein is preferably selected from the group consisting of 3,3', 5,5'-tetramethyl bezidine (TMB), 2,2'-azino-bis(3-ethylbenzothiazoline-6-sulfonic acid) (ABTS), o-phenylenediamine (OPD), diaminobenzidine (DAB), 3-amino-9-ethylcarbasole, 5-bromo-4-chloro-3-indolyl phosphate/iodonitrotetrazolium (BCIP/INT), new fuchin (NF), and fast red TR salts, but not always limited thereto.

The present invention provides a method for preparing a repeating chain-detection functional group containing the step of linking, conjugating, or fusing a detection functional group to a repeating chain of a binding domain that binds to a monomer.

The present invention also provides a method for preparing a complex of multiple monomers/repeating chain-detection functional group comprising the following steps:
1) preparing a repeating chain-detection functional group by linking, conjugating, or fusing a detection functional group to a repeating chain of a binding domain that binds to a monomer; and
2) preparing a complex of multiple monomers/repeating chain-detection functional group by mixing multiple monomers with the repeating chain-detection functional group prepared in step 1).

The present invention also provides a monomer binding domain repeating chain-detection functional group prepared by linking, conjugating, or fusing a detection functional group to a repeating chain of a binding domain that binds to a monomer.

The present invention also provides a complex of multiple monomers/repeating chain-detection functional group prepared by conjugating multiple numbers of monomers to the monomer binding domain repeating chain-detection functional group.

The present invention also provides a method for detecting a target of a monomer containing the step of forming a super-complex by mixing the multiple monomers/repeating chain-detection functional group complex of the invention with the monomer target.

In the above method, an additional step of measuring the target detection level of a monomer can be included.

In the above method, the detection functional group is preferably selected from the group consisting of Cy-3, Cy-5, FITC, GFP (green fluorescent protein), RFP (red fluorescent protein), and Texas Red, but not always limited thereto.

In the above method, in case the monomer is an antibody, the antibody is preferably selected from the group consisting of antibody fragments, Fab fragments, Fab fragment containing fragments, Fv fragments, Fv fragment containing fragments, Fc fragments, and Fc fragment containing fragments.

The present invention can be used for the production of a cancer cell specific antibody-toxin super-complex by using antibody-toxin (immunotoxin), the anticancer agent. This produced antibody-toxin super-complex has an excellent drug efficacy so that the delivery of a large volume of cell killing functional groups to a target cancer cell will be possible, by which positive cancer treatment effect is expected.

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Example 1: Preparation of repeating chains GR8 ~ GR20, GR2-2, 2-3, 2-4 of protein G antibody binding domain III

Protein G domain III gene was obtained from chromosomal DNA of KCTC 3098 distributed from Korean Collection for Type Cultures (KCTC). The plasmids pGR2-2 ~ pGR2-4 wherein G4S linker was repeated 2 ~ 4 times between two domain IIIs were constructed. The plasmid pGR1 (Y. Lee et al, Enhanced Formation of Disulfide-bridged Dimer(Fab-PE38)2 Utilizing Repeats of the Fab Binding Domain of Protein G (2010) J. Biol. Chem. 285, 5127-5131) containing protein G domain III was inserted in the additional Agel restriction enzyme site in front of the G4S linker at the end of the domain III by using site-directed mutagenesis, resulting in the construction of pGR1-A.

Quick-change site-directed mutagenesis was performed with two primers P3 [5'-AGACCTTTAC GGTAACTCAA ACCGGTGGAG GCGGGTCCGG ATA-3' (SEQ. ID. NO: 1)] and P4 [5'-TATCCGGACC CGCCTCCACC GGTTTCAGTT ACCGTAAAGG TCT-3' (SEQ. ID. NO: 2)]. After the mutagenesis, the nucleic acid sequence of pGR1-A was confirmed by dedeoxy DNA sequencing. The plasmid pGR1-A was digested with NdeI and BspEI and the fragments obtained thereby were purified. Then, pGR1 was digested with AgeI located behind Ndel site and 6 His tag site. PGR2-A was constructed by the ligation of two fragments; the big fragment obtained by digesting pGR1 with NdeI and AgeI and the small fragment obtained by digesting pGR1-A with NdeI and BspEI. And the resultant pGR2-A was digested with NdeI and AgeI to obtain a big fragment still harboring the second domain III of protein G and G4S linker in front of that. This big fragment was ligated with the small fragment obtained by digesting pGR1-A with NdeI and BspEI, resulting in the construction of pGR2-2 containing two G4S linkers in between the first domain III and the second domain III. Further, pGR2-3 having three G4S linkers and pGR2-4 having 4 G4S linkers were constructed by the same manner as used for the construction of the above pGR2-2.

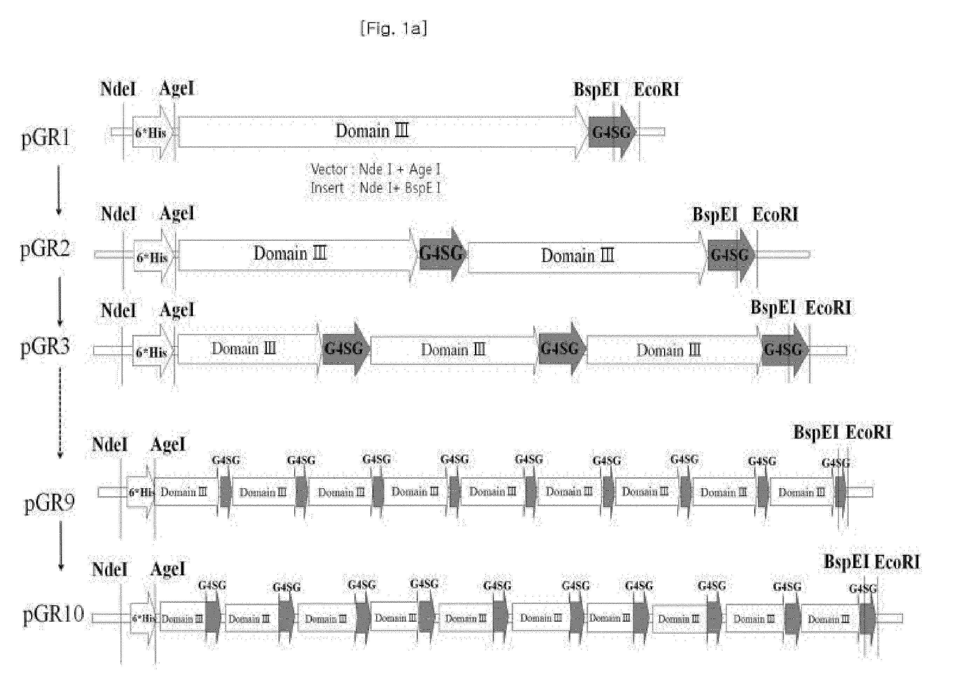

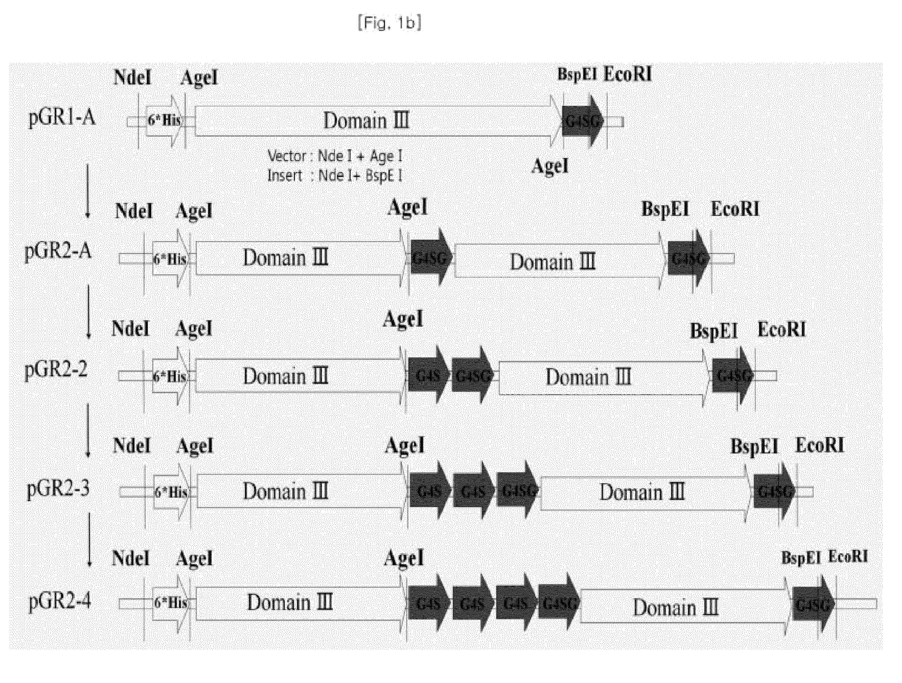

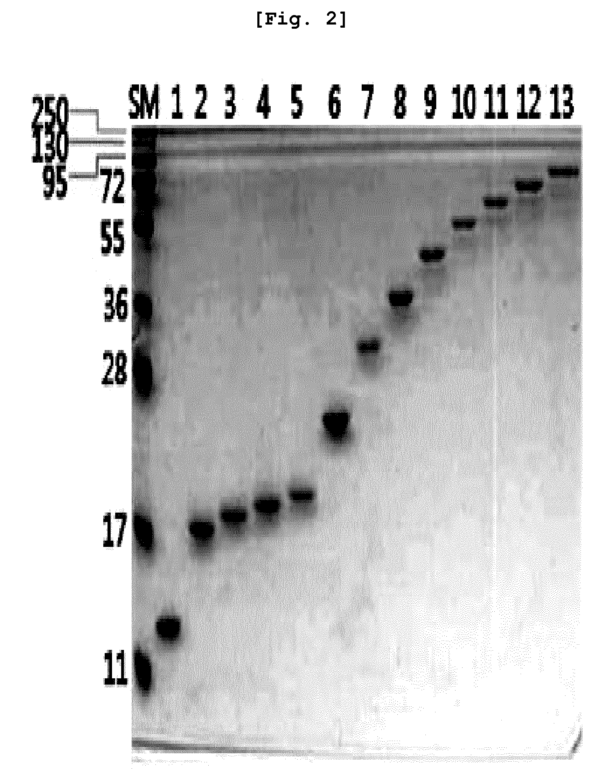

GR8 ~ GR20 were prepared by the method described in the paper of pGR1 (Y. Lee et al, Enhanced Formation of Disulfide-bridged Dimer(Fab-PE38)2 Utilizing Repeats of the Fab Binding Domain of Protein G (2010) J. Biol. Chem. 285, 5127-5131). The construction of an expression plasmid was performed by the method shown in Figure 1 and the purified repeating chain was analyzed by 16% SDS-PAGE (Figure 2).

### Example 2: Size-exclusion chromatography with the complex of Fab-toxin monomer and protein G domain III repeating chains GR1 ~ GR10, GR2-2, 2-3, and 2-4 and the formation of disulfide-bridged dimer in the complex

Protein G domain III that binds to immunoglobulin can bind to IgG Fc and Fab fragments. Protein G immunoglobulin binding site (domain III) is known to bind to Fab fragment CH1, according to the previous reports. The second β-strand of protein G domain III binds to the seventh β-strand of Fab CH1 domain in anti-parallel position. The β/β interaction between these two proteins allows five hydrogen bonds between CH1 domain and domain III. Besides, three other hydrogen bonds can also be found among major atoms of CH1 domain. Despite these two proteins form a complex, no changes in the structure of domain III or CH1 domain are found.

In this invention, two different types of repeating chains having the repeats of domain III were prepared. First, the repeating chain having one G4S amino acid between domain IIIs was prepared. The repeating chain wherein domain IIIs were repeated 10 times therein was used for the association with Fab-PE38 monomer to form a complex. The repeating chains used herein were GR1 ~ GR10. When this type of repeating chain was associated with a monomer, the complex comprising multiple numbers of monomers (2, 3, or 4 monomers) could be prepared.

The present inventors also prepared the repeating chain having G4S linkers in different lengths between two domain IIIs. There is a difference according to the linker in the association of Fab-PE38 domain and domain III. The repeating chains GR2-2, GR2-3, and GR2-4 were accordingly prepared and they had 2, 3, and 4 G4S linkers between two domain IIIs. So, two Fab-PE38 monomers were linked to a repeating chain to form a complex and as a result the length of a dimer of the monomers could be regulated.

The complexes formed by a noncovalent bond with GR2-2, GR2-3, and GR2-4 were analyzed by size exclusion column chromatography.

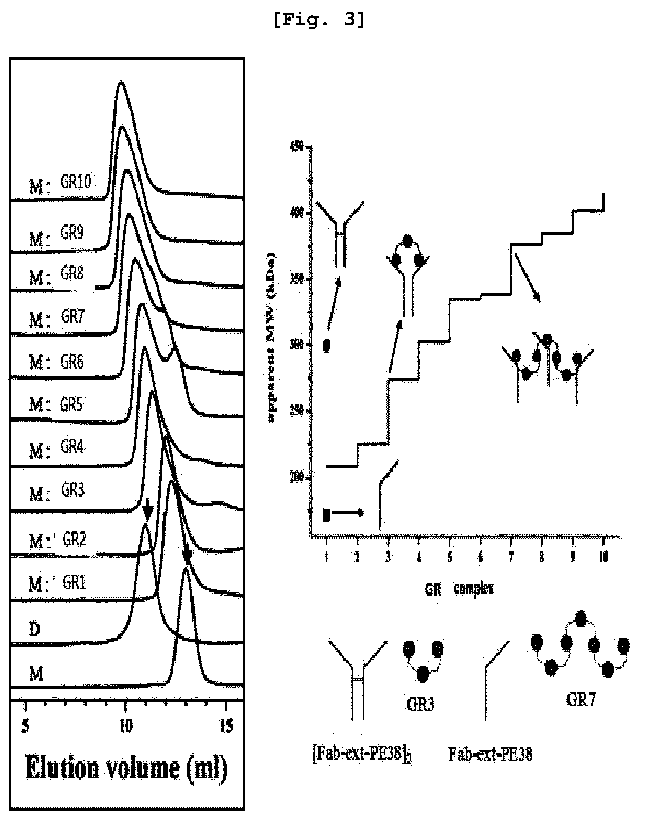

The results of size-exclusion chromatography with GR series complexes are shown in Figure 3. The size-exclusion chromatography was performed with Superdex-200 TM HR column. The association of Fab-ext-PE38 with GR series was performed at different ratios. 715 µg of Fab-ext-PE38 was associated with each GR protein. The association of Fab-ext-PE38 with GR1 ~ GR3 was performed at the molar ratio of 2:1. 715 µg of Fab-ext-PE38 was associated with 28 *µ*g of GR4 ~ GR10. All the chromatograms were presented as over-lapped according to the elution volume. Chromatograms of [Fab-ext-PE38]2(D) and Fab-ext-PE38(M), used as the standard materials, were obtained by the elution under the same condition.

The results of size-exclusion chromatography with GR2-2, 2-3, and 2-4 are shown in Figure 4.

The results of the investigation of the complex of [Fab-ext-PE38]2 and Fab-ext-PE38 mixture with GR2 or GR3 are shown in Figure 5.

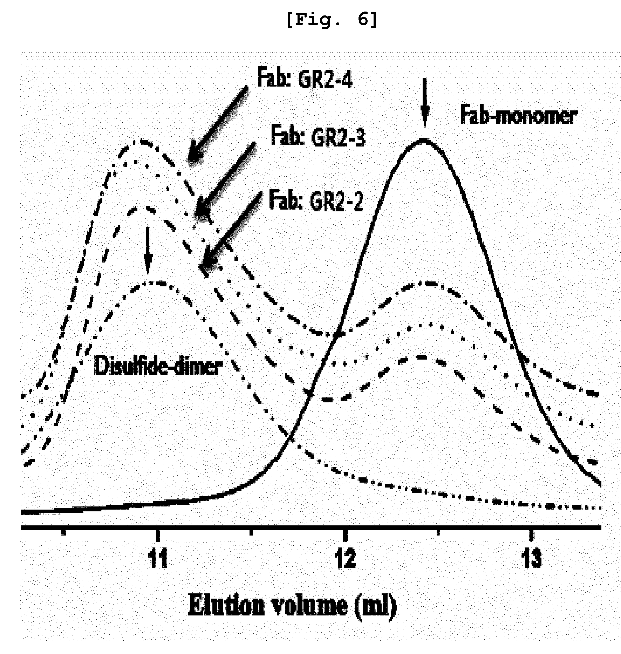

The results of size-exclusion chromatography with the complex of Fab-PE38 monomer and GR2-2, 2-3, and 2-4 are shown in Figure 6.

The results of size-exclusion chromatography for the purification of the complex of GR repeating chain and Fab-PE38 protein are shown in Figure 7.

The results of 8% non-reducing SDS-PAGE confirming the formation of disulfide-bridged dimer from Fab-toxin monomer by reduction/oxidation mixed reaction in the complex of Fab-toxin monomer and GR10 or GR2-2, GR2-3, and GR2-4 are shown in Figure 8 (Figures 3 ~ 8).

As a result, it was confirmed that two Fab-PE38 monomers could form complexes with a repeating chain respectively with forming a dimer as well.

**[Table 1]**

| Ratio of the disulfide-bridged dimer formed by reduction/oxidation mixed reaction from Fab-toxin monomer | |
|---|---|
| Antibody-toxin bound to metal | |
| chelating bead | action of disulfide dimer |
| B3(Fab)-ext-PE38] | n.d.* |
| B3(Fab)-ext-PE38]:GR1 | n.d.* |
| B3(Fab)-ext-PE38]:GR2 | n.d.* |
| B3(Fab)-ext-PE38]:GR3 | 0.7 |
| B3(Fab)-ext-PE38]:GR4 | 0.5 |
| B3(Fab)-ext-PE38]:GR5 | 0.6 |
| B3(Fab)-ext-PE38]:GR6 | 0.3 |
| B3(Fab)-ext-PE38]:GR7 | 0.6 |
| B3(Fab)-ext-PE38]:GR8 | 0.1 |
| B3(Fab)-ext-PE38]:GR9 | 0.1 |
| B3(Fab)-ext-PE38]:GR10 | 0.3 |
| B3(Fab)-ext-PE38]:GR2-2 | 0.2 |
| B3(Fab)-ext-PE38]:GR2-3 | 0.4 |
| B3(Fab)-ext-PE38]:GR2-4 | 0.3 |

| | |
|---|---|
| *n.d: not determined. | |

### Example 3: Amplification of antibody signal by protein G domain III repeating chain GR10 confirmed by Western blotting

Western blotting protocol provided by cell signaling technology^{®} was used with slight modification and the revised version of direct ELISA protocol provided by abcom^{®} was used.

Solutions and reagents used for this experiment were prepared as follows: Solutions were prepared by using water filtered with Milli-Q or water with equal purity. 1) 1X SDS sample buffer: 62.5 mM Tris-HCl (25°C, pH 6.8), 2% W/V SDS, 10 % glycerol, 50 mM DTT, 0.01 % W/V bromophenol blue or phenol red. 2) mobile buffer : 25 mM tris base, 0.2 M glycine. 3) 10X tris buffered saline (TBS) : 10X TBS 1 L: 24.2 g tris base, 80 g sodium chloride, acidity was regulated with HCl (pH 7.6) (1X). 4) ovalbumin: (weight to volume [W / V]). 5) blocking buffer: 1X TBS, 0.1% tween-20, and 2% W/V chicken serum albumin. 6) washing buffer: 1X TBS, 0.1% tween-20 (TBS/T). 7) primary antibody: anti-beta-actin mouse antibody (Santa Cruz Biotech). 8) primary antibody dilution buffer: 1X TBS, 0.1% tween-20, and 2% W/V chicken serum albumin. 9) secondary antibody: goat anti-mouse beta actin HRP. 10) blotting membrane: nitrocellulose membrane (Wattman), PVDF membrane (PALL). 11) GR recombinant protein: GR10. 12) luminal solution: 100 mM Tris/HCl pH8.8, 1.25 mM luminol, 2 mM 4IPBA, 5.3 mM hydrogen peroxide. 13) super signal femto maximum sensitivity reagent (Thermo Scientific).

Western blotting was performed as follows. A cell lysate was first prepared by using A431 or AGS cancer cell line and then the culture medium was eliminated by suction. Particularly, the cells were washed with 1X PBS and then PBS was also eliminated by suction. Then, 1X SDS sample buffer was added thereto in order to lyse the cells, followed by heating at 95 ~ 100 °C for 5 minutes. Centrifugation was performed for 5 minutes to precipitate the sample. Then, the precipitate was placed on SDS-PAGE gel (10 cm x 10 cm), followed by electrophoresis. Then, the sample was transferred onto a nitrocellulose membrane or a PVDF membrane.

Blocking the membrane surface and antibody binding reaction were performed as follows. The volume of solution was adjusted to fit the size of the membrane which was 10 cm x 10 cm (100 cm²). The volume was regulated according to the size of membranes.

For the membrane surface blocking, the nitrocellulose or PVDF membrane was washed with a proper volume of TBS at room temperature for 5 minutes after the transfer. The membrane was loaded in a proper volume of membrane surface blocking buffer, which was warmed up at room temperature for 1 hour. The membrane was washed with TBS/T for 5 minutes three times. Then, the super-complex was prepared. Particularly, GR10 was mixed with the primary antibody according to the molar ratio listed in the shown result, followed by warming at 37 °C for 1 hour. The produced super-complex was placed in ice and stored as in ice until it would be used. Then, 10 mℓ of primary antibody dilution buffer was mixed with the membrane/primary antibody complex or the primary antibody/repeating chain complex at the dilution ratio listed in the shown result in order to induce primary antibody reaction, followed by warming at room temperature for 1 hour. The mixture was washed with 15 mℓ of TBS/T three times for 5 minutes each. HRP-conjugated secondary antibody (1:2000) which had been properly selected to match the primary antibody was mixed with the membrane surface blocking buffer at a proper volume, followed by stirring softly for 1 hour at room temperature to keep it warm. The mixture was washed with 15 mℓ of TBS/T three times for 5 minutes each. Detection in section D was performed. Lastly, for the detection of an antigen protein, luminol solution or super signal femto maximum sensitivity reagent (Thermo Scientific) was mixed with the membrane, which was softly stirred at room temperature to keep it warm. The excessive developing solution was taken away with leaving it not to dry and then the membrane was rapped with plastic lab, followed by exposure on X-ray film.

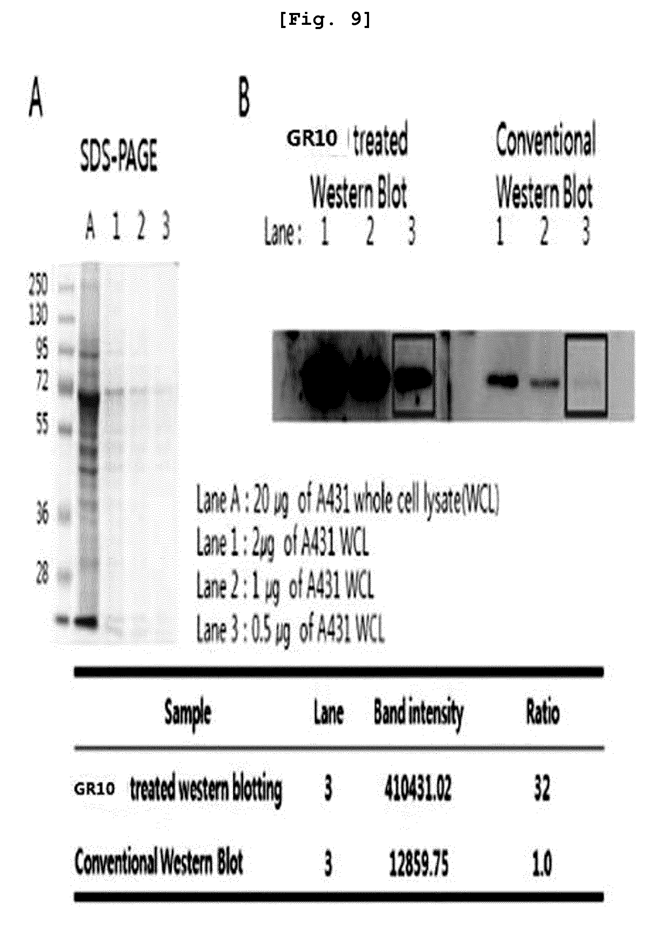

The results of the chemiluminescence signal amplification by GR10, confirmed by Western blotting, are shown in Figure 9 (Figure 9).

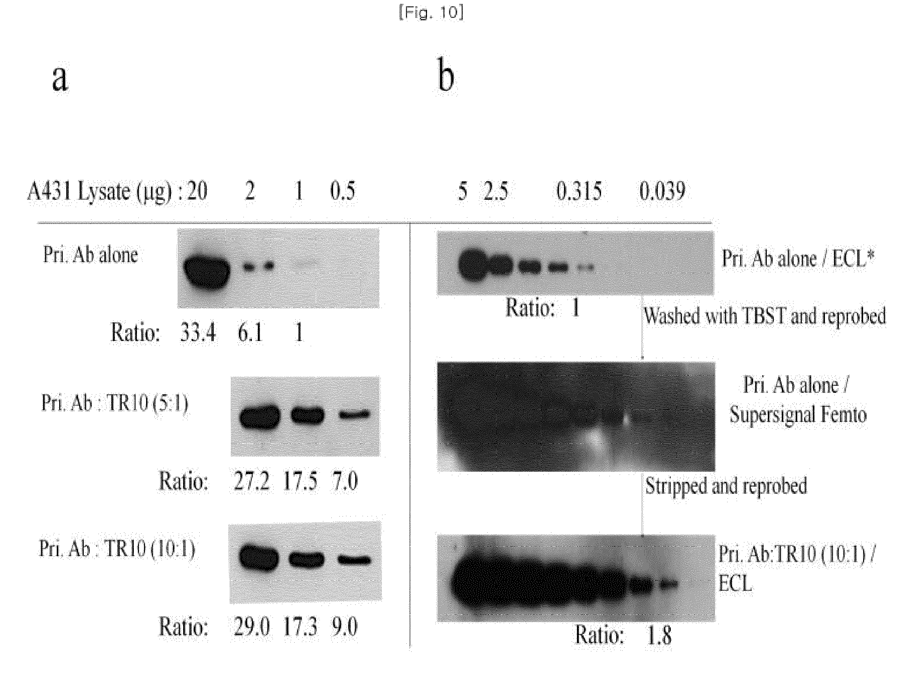

The results of the chemiluminescence signal amplification by GR10 repeating chain, confirmed by Western blotting, are shown in Figure 10 (Figure 10). Particularly, the super-complex of GR10 and the primary antibody was prepared according to the molar ratio shown in the bracket in order to form a super-complex. The primary antibody was diluted at the ratio of 1:1000 and the super-complex was prepared at the same concentration as that of the primary antibody. The goat anti-mouse-HRP conjugated antibody was used as the secondary antibody for both experiments. The primary and the secondary antibodies were all kept warm at room temperature for 1 hour. All the cell lysate samples were separated by 10% denaturing SDS-PAGE, which were then transferred onto nitrocellulose membrane. This membrane was then cut into three pieces, which were detected with the primary antibody. Themo Supersignal Femto substrate displaying a high sensitivity was used as the fluorescence color developing reagent. A431 clear cell lysate was separated by 10% denaturing SDS-PAGE and then transferred onto PVDF membrane in order to investigate whether or not the resultant super-complex could give as much signal amplification effect as nitrocellulose membrane. The membrane was first detected only with the primary antibody and then color development was induced by using the secondary antibody and the conventional ECL reagent. The membrane was washed with TBST solution, followed by inducing color development by using Supersignal Femto substrate. As a result, a very strong background noise signal was detected along with the increase of the regular signal. This background noise signal was attributed to the non-specific conjugation of the secondary antibody to PVDF membrane and the high sensitivity of Supersignal femto substrate. Thereafter, the antibodies were peeled off from the membrane by using SDS and 2-mercaptoethanol. The super-complex prepared by mixing the primary antibody and the repeating chain was linked thereto, followed by detection by using the secondary antibody and the conventional ECL color developing reagent. At this time, the super-complex demonstrated 15 times higher sensitivity than the conventional one, which was consistent result with the one observed on nitrocellulose membrane. In this experiment, the conventional chemiluminescence reagent displaying a medium level of sensitivity was used and the ECL reagent prepared by the method of Haan and Behmann (2007) was used. The signal amplification effect was similar to that observed on the nitrocellulose membrane and the amplified signal was as high as when Supersignal Femto substrate was used. However, this time the background noise signal was not as increased as before, suggesting that the signal amplification this time yielded clearer signals. A cell lysate tends to attach on PVDF easily. So, the color developing reagent displaying a medium level of sensitivity was good enough to detect a small amount of sample.

### Example 4: Amplification of antibody signal by repeating chain GR10 of protein G domain III confirmed by enzyme-linked immunosorbent assay (ELISA)

Indirect ELISA was performed as follows.

Solutions and reagents used for this experiment were prepared as follows: Solutions were prepared by using water filtered with Milli-Q or water with equal purity. 1) bicarbonate/carbonate coating buffer (100 mM), Antigen or antibody was diluted in a coating buffer for fixation in wells: 3.03 g of Na₂CO₃, 6.0 g of NaHCO₃, 1000 mℓ of distilled water (pH 9.6). 2) PBS. 3) blocking buffer: PBS containing 1% BSA serum. 4) washing buffer: PBS containing 0.05% (v/v) Tween 20. 5) antibody dilution buffer: Antibodies were diluted in 1X blocking buffer in order to reduce nonspecific binding of the primary and secondary antibodies.

For the antigen coating on a microplate, A431 or AGS cell line was used for the preparation of a cell lysate. The cell lysate was diluted with carbonate coating buffer to make the final concentration 20 µg/mℓ. Then, 50 µℓ of the diluted cell lysate was distributed on the top of the micro-plate well, followed by coating. The plate was covered and stayed at 4 °C for overnight. The coating buffer was eliminated and each well was washed with 200 *µℓ* of PBS. The plate was turned upside down and shaken in the sink in order to eliminate the used coating buffer or washing buffer. The remaining solution was eliminated with paper towel by tapping softly.

For the membrane surface blocking, 200 µℓ of 1% BSA/PBS blocking buffer was added to each well of the plate to block the protein binding site that remained uncoated in the coated well. Then, the plate was covered with a lid and kept warm at room temperature for at least 2 hours, followed by washing with PBS twice. To induce antibody reaction, 100 *µℓ* of the primary antibody diluted at the designated concentration right before being used or the super-complex was added to the blocking buffer, and then the plate was covered with a lid and kept warm at room temperature for 1 hour. Then, the plate was washed with PBS twice and 100 µℓ of the secondary antibody-HRP diluted at the optimum concentration was added to each well of the plate. The plate was covered with a lid and kept warm at room temperature for 1 hour. The plate was washed with PBS twice.

For the detection, 100 *µℓ* of TMB substrate solution was added to each well of the plate by using a multichannel pipette. When the color development was fully induced (30 minutes), 100 µℓ of stop solution was added to each well of the plate. Absorbance (optical density) of each well was measured by using a plate reader.

As a result, the sensitivity of ELISA was significantly increased by GR10, which is shown in Figure 11 (Figure 11). Precisely, two different ELISAs were performed. The significant amplification of ELISA signal was observed when the super-complex of monoclonal anti-β-actin antibody and GR10 was used as the primary antibody. AGS cell lysate was placed in a general 96-well cell culture plate, which stayed at 4 °C for overnight. ELISA was performed according to the standard protocol. The super-complex of GR10 and the primary antibody was prepared according to the molar ratio shown in the bracket. The primary antibody used herein was the mouse monoclonal anti-β-actin antibody. The goat anti-mouse-HRP conjugated antibody was used as the secondary antibody for both experiments. Both the primary and the secondary antibodies were reacted at room temperature for 1 hour. The substrate TMB was reacted at room temperature for 30 minutes.

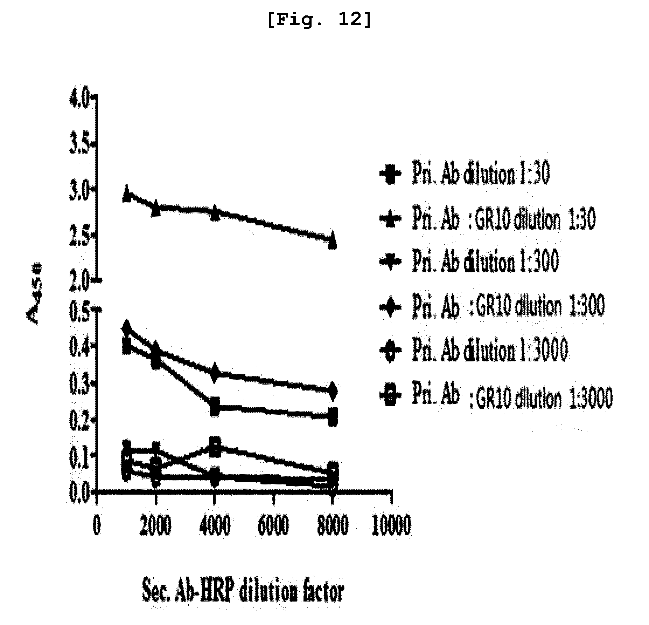

The signal amplification effect according to the serial dilution of the secondary antibody is shown in Figure 12. ELISA signal was significantly increased by the super-complex of the primary antibody and GR10. The signal was quite regular in the tested dilution range of the secondary antibody. When the super-complex of the mouse monoclonal anti-β-actin antibody and GR10 was used as the primary antibody for the above ELISA, a significant signal amplification was observed. At this time, A431 cell lysate was used. The complex of GR10 and the primary antibody was prepared according to the molar ratio of 1:10. The goat anti-mouse HRP conjugate was used as the secondary antibody. Each well was coated with 1 g of A431 cell lysate. The primary antibody was serially diluted 10-fold. The primary antibody was diluted according to the designated dilution ratio and the secondary antibody was serially diluted 2-fold.

### Example 5: Increase of sensitivity of influenza rapid antigen test kit by repeating chain GR10 of protein G domain III

The increase of sensitivity of influenza rapid antigen test kit, the most representative diagnostic kit using an antibody, by GR10 was observed. SD Bioline influenza antigen rapid test kit or the kit provided by Korea Green Cross Co was used herein. The kit included antigen buffer, dropper, tube, cotton swab for the collection of sample, and strip. The antigen buffer was sucked up to the dropper line and then the antigen buffer was placed in the tube. An antigen sample was loaded in the tube containing the antigen buffer, followed by well-mixing at least 5 times. At this time, GR10 protein was simply diluted with an antigen in the antigen buffer. The strip was added into the tube, which was read 10 ~ 15 minutes later.

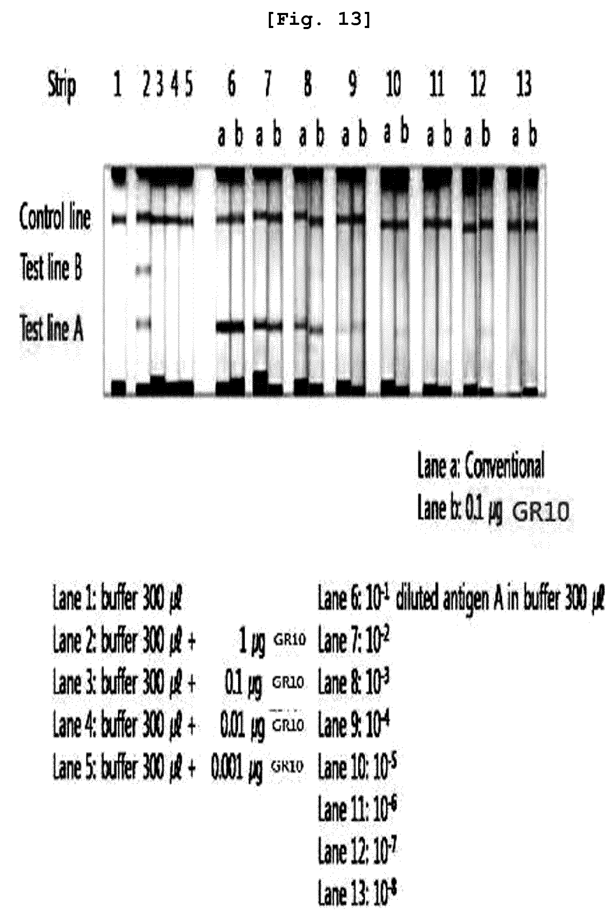

As a result, when GR10 protein was simply diluted together with an antigen in the conventional rapid antigen test kit antigen dilution buffer, the antigen could be detected until it was 1000-times diluted, unlike in the absence of GR10 (Figure 13).

### Example 6: Immunofluorescence with a cancer cell line using GR protein as the antibody labeling reagent

GR1 was conjugated with fluorescein isothiocyanate (FITC), the fluorochrome, and the conjugated GR1-FITC was used for immunofluorescent staining of A431 (human squamous carcinoma), the cultured cancer cell line. Mouse anti-LC3 antibody was used as the primary antibody. The prepared GR1-FITC was used instead of the secondary antibody for the detection. The cells detected by fluorescence were stained with F-actin specific rhodamine-phalloidin (Sigma Aldrich). The color development was observed under fluorescent microscope.

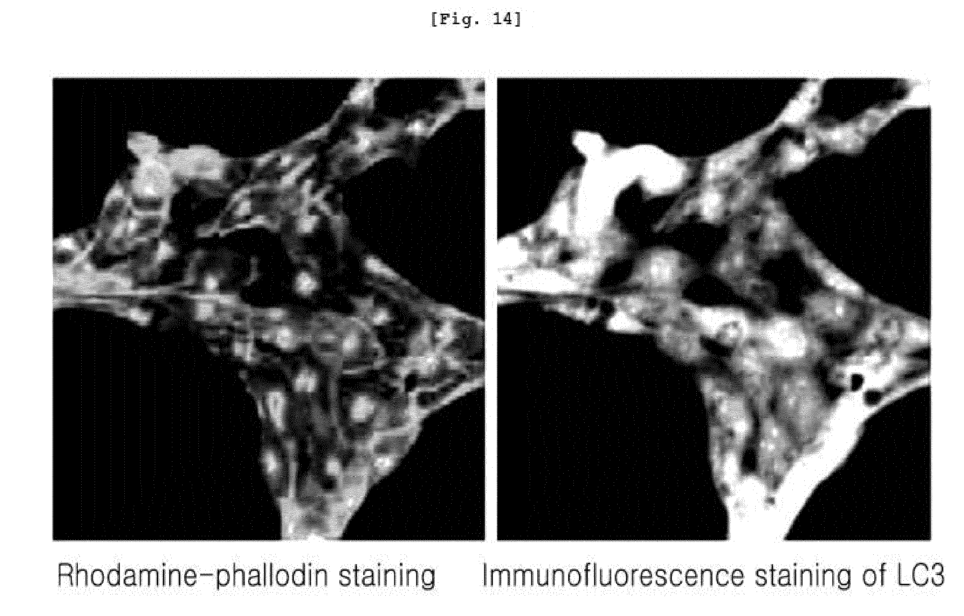

As a result, compared with when the secondary antibody-fluorochrome conjugate was used, much smaller amount of protein could be detected by using the GR1-FITC conjugate. Clear immunofluorescent staining images could be obtained (Figure 14).

Therefore, like the GR1-FITC, GR protein can be linked, conjugated, or fused with a detection functional group, and the resultant conjugate can be usable for any kinds of antibodies and thus there is no need to conjugate a detection functional group to each antibody. GR protein is a very small molecule, so that the production of GR protein is easy and the handling thereof is also simple and easy.

### Example 7: Construction of the plasmid expressing repeating chain of Staphylococcus aureus protein A antibody binding domain B

The plasmid prepared by introducing the synthesized protein A domain B (Table 1) DNA sequence into a vector was distributed from Bioneer Corporation. *E. coli* DH5α was transfected with the plasmid and cloned.

The said plasmid was digested with NdeI and BspEI. The resultant small DNA fragment (245 bp) was purified. The DNA fragment was cloned into pGR1 (pTR1) vector which was digested with the same enzymes, resulting in the construction of pAR1 containing one protein A domain B. Then, DNA sequence of the above domain B was confirmed by dideoxy DNA sequencing.

There is (G4S)2 sequence composed of 10 amino acids as a spacer between each domain B. To construct pAR2 wherein protein A domain B repeats two times, the pAR1 (Table 2) constructed above was digested with NdeI and BspEI. Then, the small DNA fragment (245 bp) encoding two G4Ss and domain B was conjugated to the big fragment of the same plasmid digested with NdeI and AgeI. The resultant pAR2 was digested with NdeI and AgeI. The obtained big fragment was conjugated to the small fragment (245 bp), resulting in the construction of pAR3 wherein protein A domain B repeats three times. By the same cloning method as described above, pAR5 wherein protein A domain B repeats 5 times has been constructed.

**[Table 2]**

| Nucleotide sequence of protein A domain B |
|---|
| 5'- CATATGCATCATCATCATCACCACACCGGTTCTCAAGCCCCAAAAGCCGACAA TAAATTTAATAAAGAGCAGCAGAACGCGTTTTATGAAATCTTGCATCTGCCGA ATCTGAATGAAGAACAACGTAACGGATTCATTCAGAGCCTTAAAGATGATCCT AGTCAGTCCGCTAACTTACTCGCAGAAGCTAAGAAACTGAATGATGCACAGGC GCCGAAGGGAGGGGGTGGATCCGGTGGTGGCGGCTCCGGATAAGAATTC- 3'(SEQ. ID. NO: 3) |

| |
|---|
| * underlined part: coding sequence |

**[Table 3]**

| Plasmid and protein used in this invention | | |
|---|---|---|
| Plasmid | Protein | Reference |
| pLR1~5 | LR1~5: (His)₆- (B1-G₄S-G₄S)ₙ, n=1~5. ^{a, b, c.} | This invention |

| | | |
|---|---|---|
| ^{a}G4S: amino acid sequence of GGGGS (SEQ. ID. NO: 4) ; ^{b} (His) 6: 6x histidine tag; and ^{c}B: Staphylococcus aureus protein A domain B. | | |

The protein was over-expressed from the repeating chain construct via the conventional method reported previously (J.H. Park, et al., Mol Cells 12 (2001) 398-402).

The pure lysate was separated by Ni²⁺-chelating sepharose fast flow chromatography (Amersham Bioscience, Sweden), followed by size-exclusion chromatography using Hiload Superdex-75 pg or Hiload Superdex-200 pg(26/60) (Amersham Bioscience, Sweden).

### Example 8: Construction of the plasmid expressing repeating chain of Peptostreptococcus magnus protein L antibody binding domain B1

The plasmid prepared by introducing the synthesized protein L domain B1 DNA sequence into a vector was distributed from Bioneer Corporation. *E. coli* DH5α was transfected with the plasmid and cloned.

The said plasmid was digested with NdeI and BspEI. The resultant small DNA fragment (299 bp) was purified. The DNA fragment was cloned into pGR1 (pTR1) vector which was digested with the same enzymes, resulting in the construction of pLR1 containing one protein L domain B1. Then, DNA sequence of the above domain B1 was confirmed by dideoxy DNA sequencing.

There is (G4S)2 sequence composed of 10 amino acids as a spacer between each domain B1. To construct pLR2 wherein protein L domain B1 repeats two times, the pLR1 (Table 5) constructed above was digested with NdeI and BspEI. Then, the small DNA fragment (299 bp) encoding two G4Ss and domain B1 was conjugated to the big fragment of the same plasmid digested with NdeI and AgeI. The resultant pLR2 was digested with NdeI and AgeI. The obtained big fragment was conjugated to the small fragment (299 bp), resulting in the construction of pLR3 wherein protein L domain B1 repeats three times. By the same cloning method as described above, pLR5 wherein protein L domain B1 repeats 5 times has been constructed.

**[Table 4]**

| Nucleotide sequence of protein L domain B1 |
|---|
| Nucleotide sequence of protein L domain B1 |
| 5'- CATATGCATCACCATCACCATCATACCGGTATCAAGTTCGCCGGTAAAGAAGAAACGC CGGAAACCCCTGAGACAGACAGTGAAGAGGAAGTGACAATAAAAGCAAATCTGATTTT CGCCAACGGGTCAACCCAGACGGCCGAATTCAAAGGGACATTTGAAAAAGCAACTTCT GAGGCTTATGCATACGCGGACACTCTGAAGAAGGATAATGGTGAATATACCGTAGATG TTGCTGATAAAGGTTATACCCTGAATATTAAATTTGCGGGTGGCGGCGGCGGAAGCGG TGGCGGAGGTTCCGGATAAGAATTC-3'(SEQ. ID. NO: 5) |

| |
|---|
| * underlined part: coding sequence |

**[Table 5]**

| Plasmid and protein used in this invention | | |
|---|---|---|
| Plasmid | Protein | Reference |
| pLR1~5 | LR1~5: (His)₆-(B1-G₄S-G₄S)ₙ, | This invention |
| | n=1~5. ^{a, b, c.} | |

| | | |
|---|---|---|
| ^{a}G4S: amino acid sequence of GGGGS (SEQ. ID. NO: 4) ; ^{b} (His) 6: 6x histidine tag; and ^{c}B1: Peptostreptococcus protein L domain B1. | | |

The protein was over-expressed from the repeating chain construct via the conventional method reported previously (J.H. Park, et al., Mol Cells 12 (2001) 398-402).

The pure lysate was separated by Ni²⁺-chelating sepharose fast flow chromatography (Amersham Bioscience, Sweden), followed by size-exclusion chromatography using Hiload Superdex-75 pg or Hiload Superdex-200 pg(26/60) (Amersham Bioscience, Sweden).

### Example 9: Construction of the plasmid expressing repeating chain of Peptostreptococcus magnus protein L antibody binding domain B1 and Staphylococcus aureus protein A antibody binding domain B

The plasmid prepared by introducing the synthesized protein L domain B1 DNA sequence into a vector was distributed from Bioneer Corporation for the construction of pLR1.

The said plasmid pLR1 was digested with NdeI and BspEI. The resultant small DNA fragment (299 bp) was purified. The DNA fragment was cloned into pAR1 (including protein A domain B) vector which was digested with the same enzymes, resulting in the construction of pLAR1 containing one protein L domain B1 linked to one protein A domain B (B1-B). Then, DNA sequences of the above domain B1 (protein L domain B1) and domain B (protein A domain B) were confirmed by dideoxy DNA sequencing.

There is (G4S)2 sequence composed of 10 amino acids as a spacer between each domain B1 (protein L domain B1) and domain B (protein A domain B). To construct pLAR2 wherein LA sequence (B1-B, protein L domain B1 - protein A domain B) repeats two times, the plasmid pLAR1 (Table 6) obtained above was digested with NdeI and BspEI. Then, the small DNA fragment (521 bp) encoding B1-(G4S)2-B-(G4S)2 (B1-B) was conjugated to the big fragment of the same plasmid digested with NdeI and AgeI. The resultant pLAR2 was digested with NdeI and AgeI. The obtained big fragment was conjugated to the small fragment (521 bp), resulting in the construction of pLAR3 wherein LA sequence repeats three times. By the same cloning method as described above, pLAR3 wherein protein L domain B1 - protein A domain B sequence repeats 3 times has been constructed.

**[Table 6]**

| Nucleotide sequence of protein LA domain B1-B |
|---|
| Nucleotide sequence of protein LA domain B1-B |
| 5'- CATATGCATCACCATCACCATCATACCGGTATCAAGTTCGCCGGTAAAGAAGAAACGC CGGAAACCCCTGAGACAGACAGTGAAGAGGAAGTGACAATAAAAGCAAATCTGATTTT CGCCAACGGGTCAACCCAGACGGCCGAATTCAAAGGGACATTTGAAAAAGCAACTTCT GAGGCTTATGCATACGCGGACACTCTGAAGAAGGATAATGGTGAATATACCGTAGATG TTGCTGATAAAGGTTATACCCTGAATATTAAATTTGCGGGTGGCGGCGGCGGAAGCGG TGGCGGAGGTTCCGGTTCTCAAGCCCCAAAAGCCGACAATAAATTTAATAAAGAGCAG CAGAACGCGTTTTATGAAATCTTGCATCTGCCGAATCTGAATGAAGAACAACGTAACG GATTCATTCAGAGCCTTAAAGATGATCCTAGTCAGTCCGCTAACTTACTCGCAGAAGC TAAGAAACTGAATGATGCACAGGCGCCGAAGGGAGGGGGTGGATCCGGTGGTGGCGGC TCCGGATAAGAATTC-3'(SEQ. ID. NO: 6) |

| |
|---|
| * underlined part: coding sequence |

**[Table 7]**

| | | |
|---|---|---|
| Plasmid and protein used in this invention | | |

| Plasmid | Protein | Reference |
|---|---|---|
| pLAR1~3 | LAR1~3: (His)₆-(B1-G₄S-G₄S-B-G₄S-G₄S)_{n,} n=1~3. ^{a, b, c, d} | This invention |

| | | |
|---|---|---|
| ^{a}G4: amino acid sequence of GGGG (SEQ. ID. NO: 7) ; ^{b} (His) 6: 6x histidine tag; ^{c}B1: Peptostreptococcus protein L domain B1; and ^{d}B: Staphylococcal protein A domain B. | | |

The repeating chain construct was over-expressed by the conventional method reported previously (J.H. Park, et al., Mol Cells 12 (2001) 398-402).

The pure lysate was separated by Ni²⁺-chelating sepharose fast flow chromatography (Amersham Bioscience, Sweden), followed by size-exclusion chromatography using Hiload Superdex-75 pg or Hiload Superdex-200 pg(26/60) (Amersham Bioscience, Sweden).

### Example 10: Investigation of cross-binding between repeating chain and gold antibody complex or super-complex and detection line antibody by using rapid antigen test

The most representative detection method using an antibody, influenza rapid antigen test kit was used to detect gold antibody by using a cross-binding between the repeating chain linked gold antibody complex or the super-complex and the detection line antibody in the absence of an antigen. The detection line generated therein was due to the cross-binding between the detection line antibody and the empty space of the antibody binding domain of the repeating chain of the repeating chain/gold antibody complex or the super complex. Herein, the influenza antigen test kit provided by SD Bioline and the influenza antigen test kit provided by Korea Green Cross Co were used. The kit included antigen buffer, cotton swab for the collection of sample, and strip. The antigen buffer was opened and the buffer was poured in a tube. A series of GR, AR, LA, and LAR repeating chain was added to the tube containing the antigen buffer, followed by mixing at least 5 times. The strip was added into the tube, which was read 10 ~ 15 minutes later.

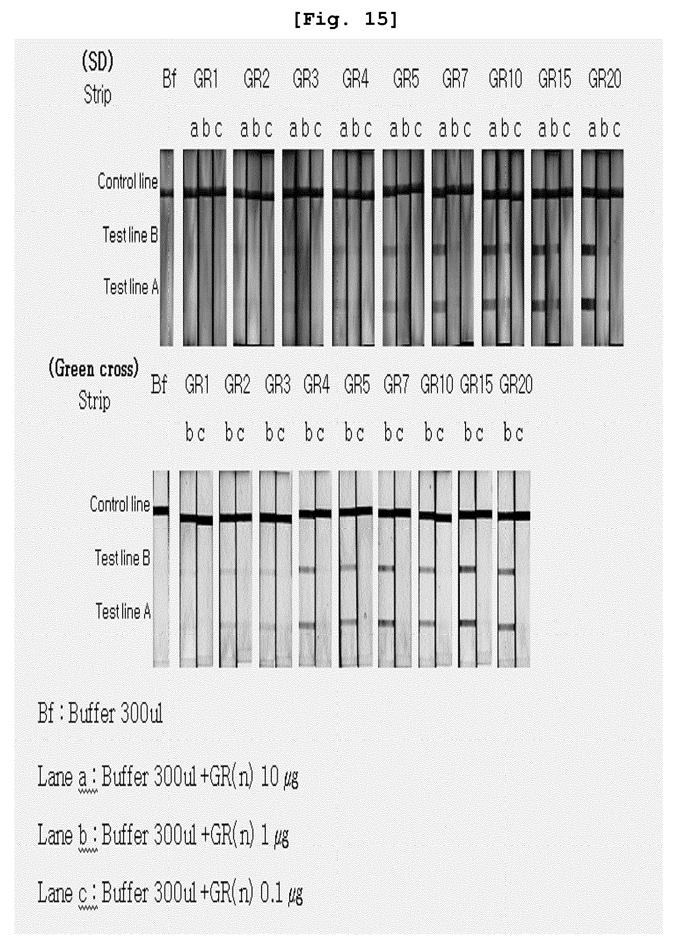

On the SD strip, when GR4, GR5, GR7, GR10, GR15, and GR20 proteins simply diluted in antigen buffer (10 *µ*g and 1 *µ*g*)* were used, a band was observed, suggesting that there was a cross-binding between them. On the Korea Green Cross strip, when 1 *µ*g of the protein was used, a stronger band was observed. However, at the concentration of 0.1 µg, both of the strips did not produce any band, suggesting that the concentration of 0.1 *µ*g was the amount of repeating chain that did not make a detection line in the absence of an antigen. This concentration of repeating chain is adequate for the examination of the effect of repeating chain on the antigen detection in the presence of an antigen (Figure 15).

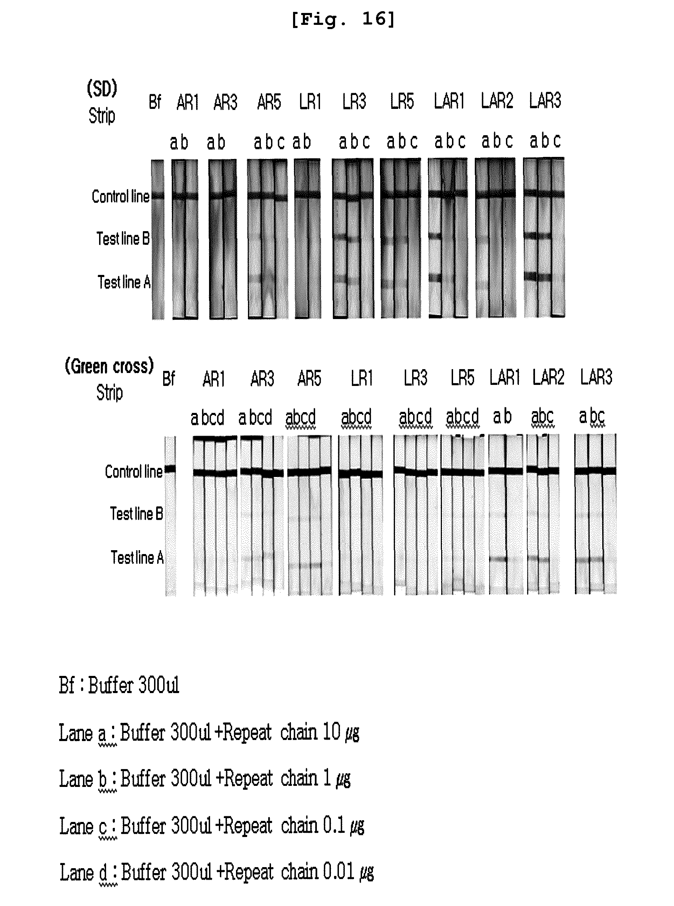

On the SD strip, when AR5, LR3, LR5, LAR1, LAR2, and LAR3 were used, the detection band generated by the gold antibody was observed. However, on the Korea Green Cross strip, no detection band was observed when LR series was used. This result indicates that the antibody included in the Korea Green Cross kit was not the one composed of kappa light chain since LR antibody binding domain only binds to kappa light chain among the light chains of antibody. At the repeating chain concentration of 0.1 *µ*g*,* neither SD nor Korea Green Cross strip produced a band, so this amount is adequate only for the experiment measuring the effect of a repeating chain on the antigen detection (Figure 16).

### Example 11: Effect of repeating chain on signal amplification in influenza rapid antigen test kit

The amplification of sensitivity of influenza rapid antigen test kit, the most representative antigen detection kit using an antibody, by GR, AR, LR, and LAR proteins was investigated. Herein, the influenza antigen test kit provided by SD Bioline was used. The kit included extraction solution, cotton swab for the collection of sample, and strip. The extraction solution was opened and the solution was poured in a tube. The antigen H1N1 was added in the tube containing the extraction solution, followed by mixing at least 5 times. At this time, GR, AR, LR, and LAR proteins were simply diluted along with an antigen in the antigen dilution buffer. The strip was added into the tube, which was read 10 ~ 15 minutes later.

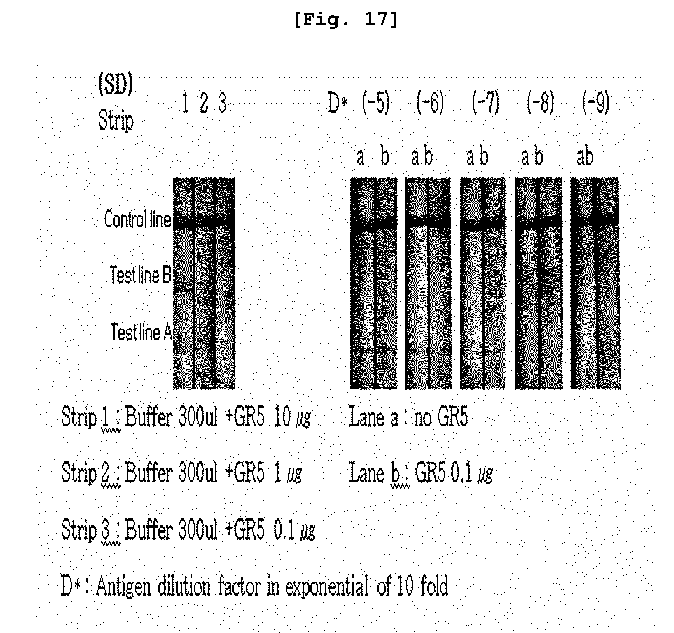

As a result, compared with when GR5 was not included, when GR5 was diluted together with the antigen, the antigen was detected until it was 10⁻⁷ times diluted (Figure 17).

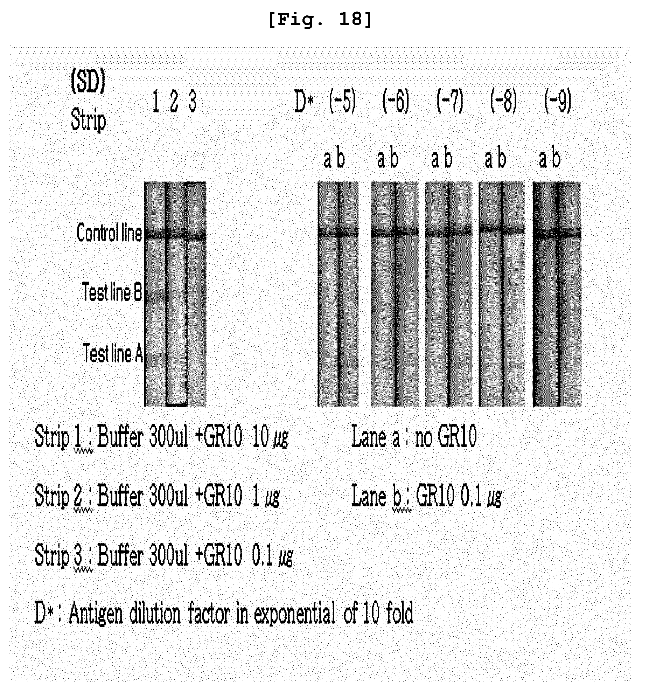

When GR10 was diluted together with the antigen, the antigen was detected until it was 10⁻⁸ times diluted, compared with when GR10 was not included (Figure 18).

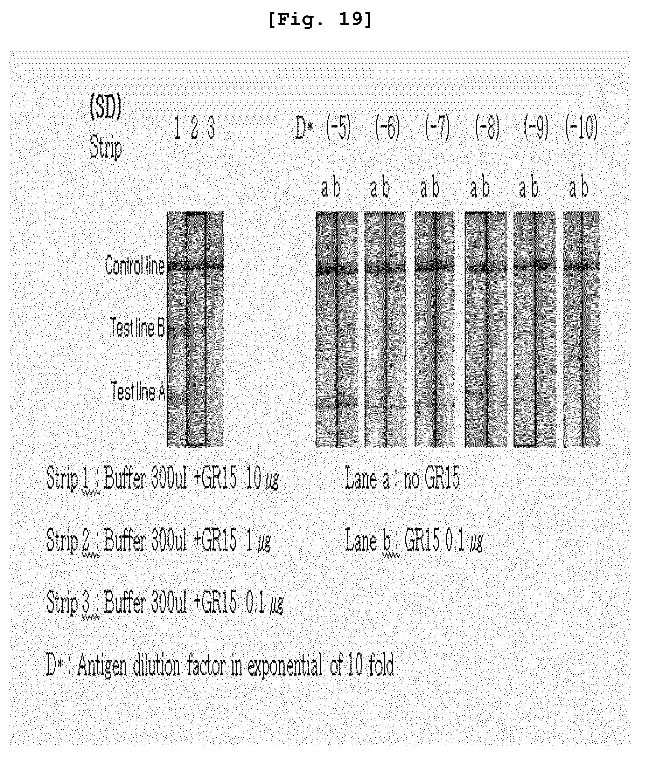

When GR15 was diluted together with the antigen, the antigen was detected until it was 10⁻⁹ times diluted, compared with when GR15 was not included (Figure 19).

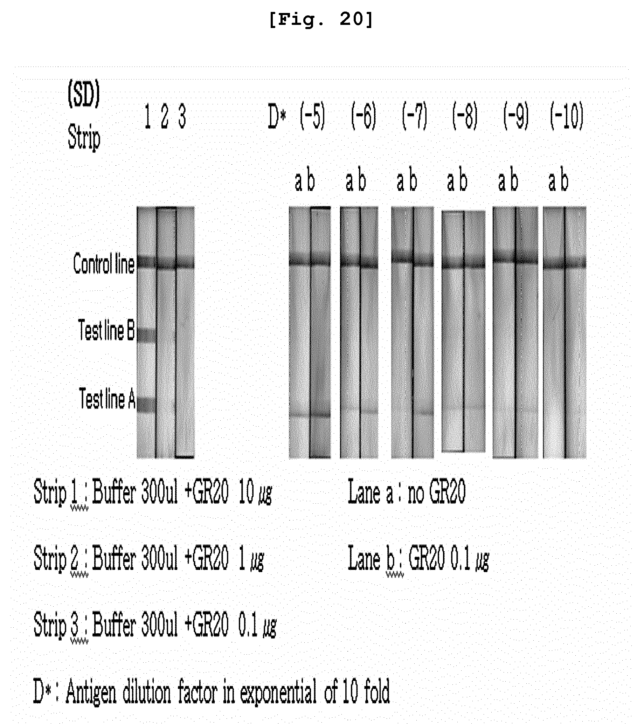

When GR20 was diluted together with the antigen, the antigen was detected until it was 10⁻¹⁰ times diluted, compared with when GR20 was not included (Figure 20).

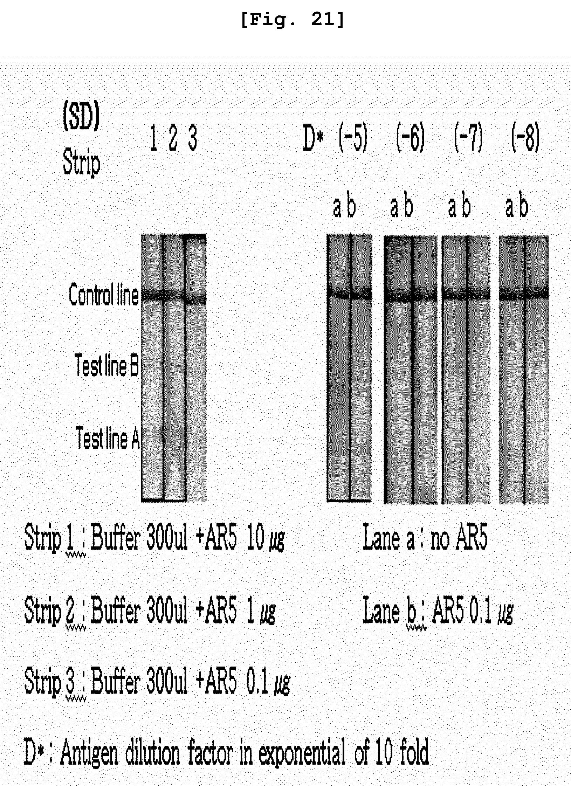

When AR5 protein was diluted together with the antigen, there was no specific effect by that, compared with when AR5 was not included (Figure 21).

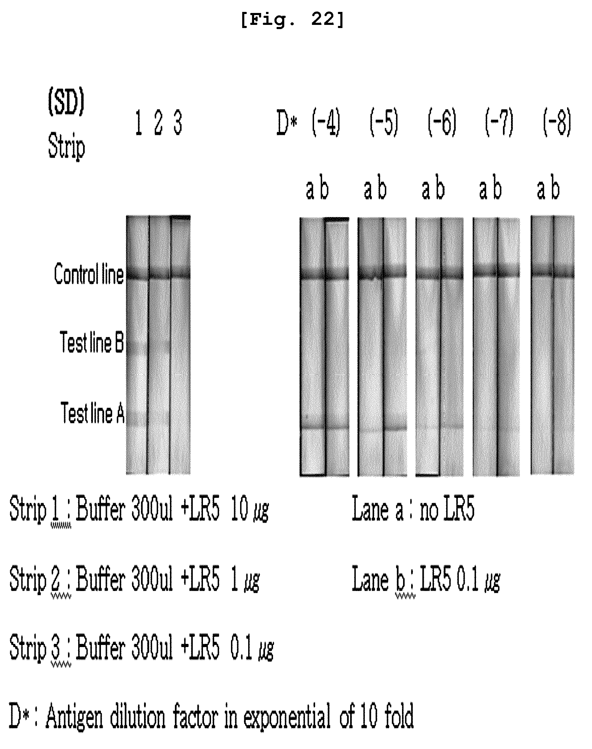

When LR5 was diluted together with the antigen, the antigen was detected until it was 10⁻⁶ times diluted, compared with when LR5 was not included (Figure 22).

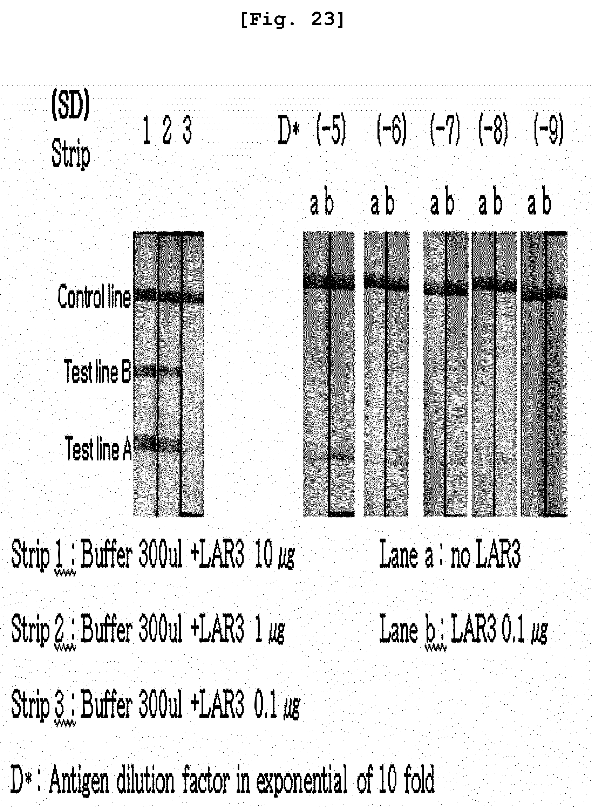

When LAR3 was diluted together with the antigen, the antigen was detected until it was 10⁻⁸ times diluted, compared with when LAR3 was not included (Figure 23).

### Example 12: Precipitation of the super-complexes of the repeating chains GR, AR, LR, and LAR and IgG

IgG solution was prepared by dissolving mouse IgG powder provided from Equitech Bio Co in PBS. The IgG solution dissolved in PBS was rocked on a rocker for 2 hours, followed by centrifugation at 21,000 rpm at 4°C for 2 hours to eliminate completely the IgG particles that were not dissolved in the solution. GR1 ~ GR20 and IgG were all quantified by BCA protein quantification method (Bicinchoninic Acid protein assay), which would be diluted at the proper concentrations for the experiment. A proper amount of GR1 ~ GR20 solution and IgG solution were mixed in a microcentrifuge tube, which stayed at room temperature for overnight for binding. Then, centrifugation was performed at 13,000 rpm at 20 °C for 30 minutes. At this time, if a super-complex was formed and precipitated, it would be observed by the naked eye. The supernatant was eliminated by using a micro-pipette. 500 µℓ of 75% ethanol was added thereto and then eliminated again, by which all the remaining solution on the wall of the microcentrifuge tube was washed off. The remaining 75% ethanol therein was eliminated by evaporation, followed by SDS-PAGE.

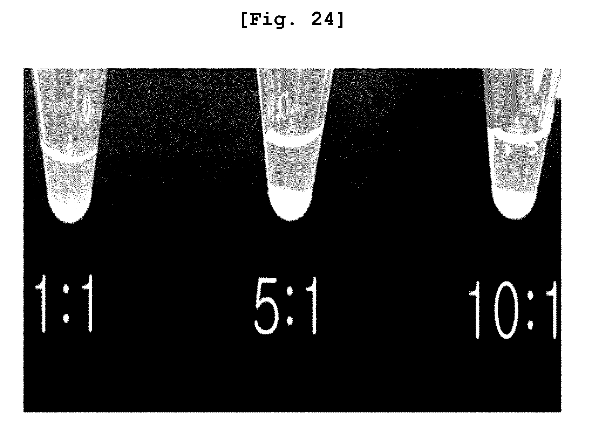

As a result, when IgG was mixed with GR10 at the molar ratio of 5:1, more precipitates were formed than when IgG was mixed with GR10 at the molar ratio of 1:1 or 10:1 (Figure 24).

After mixing IgG and GR10 at the molar ratio of 5:1, GR1 and GR2 at the same concentration as that of GR10 were reacted thereto. When GR10 was added thereto, more precipitates were formed compared with when GR1 and GR2 were used. This result was observed not only by the naked eye but also on SDS-PAGE (Figure 25). When the equal volume was added, the total mole number of the antibody binding domain DIII in the reaction solution was the same. In GR10, 10 DIIIs were repeated and linked together in one chain. In GR2, 2 DIIIs were repeated and linked together in one chain. If the total amount of GR protein added thereto was the same, the total amount of domain DIII was the same. At this time, GR10 favored the formation of a super-complex since GR10 is the longer chain than GR1 or GR2.

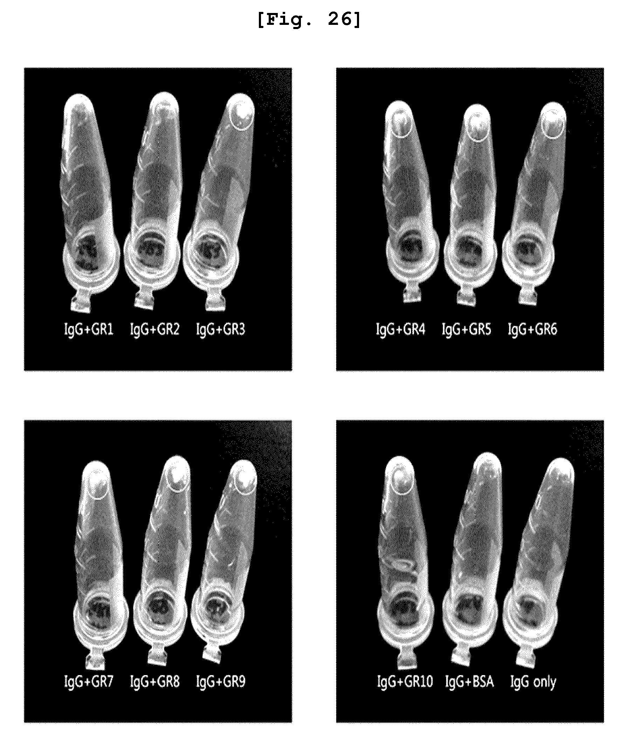

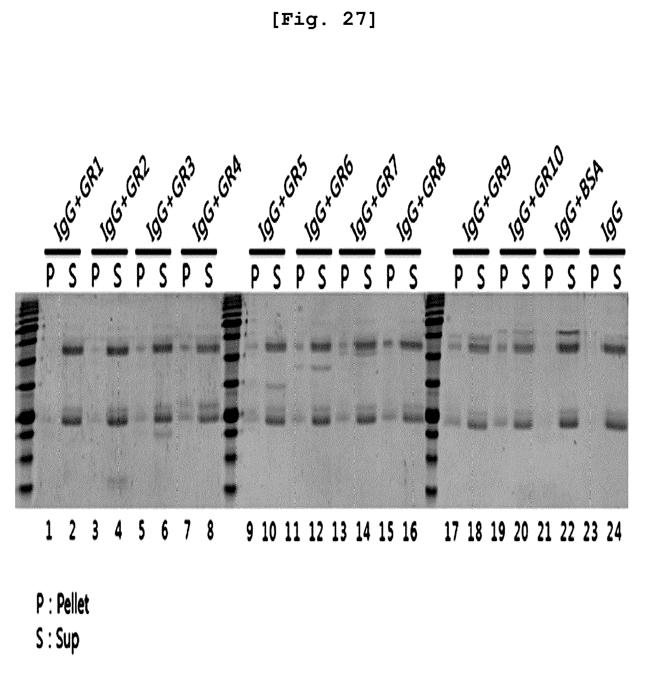

IgG was mixed with GR10 at the molar ratio of 5:1, and GR1 ~ GR9 were also mixed with IgG at the same concentration as GR10. The final concentration of IgG, after mixed with GR protein, was 400 µg/mℓ. GR3 ~ GR10 produced precipitates, observed by the naked eye (Figure 26). The consistent result was obtained by SDS-PAGE (Figure 27). From the above results, it was confirmed that a super-complex, which can be precipitated, was formed by the cross-binding between IgG and GR protein complexes.

A precipitate was not generated in the control sample containing IgG alone. A precipitate was not formed either in another control treated not with GR protein but with BSA. Therefore, it was suggested that a precipitate was not due to the decrease of protein solubility caused by the introduction of a heterologous protein but due to the formation of a super-complex with a big molecular weight resulted from the repeating chain.

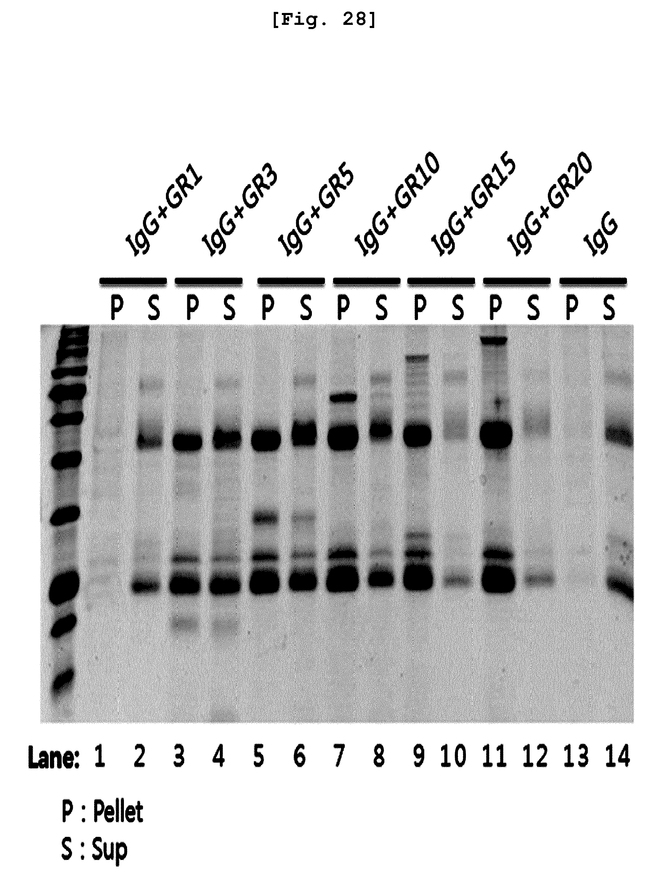

To investigate whether or not a super-complex was formed when domain DIII repeats outnumbered the number of GR10, IgG was mixed with GR10 at the molar ratio of 5:1 and then GR1, 3, 5, 15, and 20 were also mixed with IgG at the same concentration as GR10. Except the case using GR1 and IgG, precipitate was formed in all the other cases. The result of SDS-PAGE also confirmed that the complexes (GR3 ~ GR20) produced precipitates (Figure 28). So, GR15 or GR20 which is bigger than GR10 could also form a super-complex with IgG, which can be precipitated.

The same experiment was performed again with reducing the final concentration of IgG. At this time, when the final concentration of IgG was reduced lower than 25 µg/mℓ, a precipitate by GR was not observed by the naked eye. So, the formation of a precipitate resulted from the formation of a super-complex was confirmed to be dependent on the concentration of IgG. In addition to GR, AR1, 3, 5, LR1, 3, 5, LAR1, 2, and 3 were also reacted with IgG at the molar ratio of 5:1. At that time, the final concentration of IgG was 500 µg/mℓ. Unlike GR, a precipitate was not generated by AR, LR, or LAR, which suggested that these repeating chains did not bind to IgG to form an insoluble precipitate that could be observed by the naked eye.

### Example 13: Construction and expression of the plasmid prepared by the fusion between protein G antibody binding domain III repeating chain and green fluorescent protein (GFP)

GR1, GR5, and GR10 including Streptococcus protein G domain III repeating chain were fused with GFP DNA sequence (Table 8), resulting in the construction of the plasmids GR1-GFP, GR5-GFP, and GR10-GFP.

PCR primers that could over-lap the sequences of GR1 and GFP were constructed (Table 9 and Table 10). First, PCR was performed with GR1 forward and reverse primers by using GR1 as a template, and as a result PCR product 1 was obtained.

PCR was performed with GFP forward and reverse primers by using GFP as a template, and as a result PCR product 2 was obtained. PCR was performed with GR1 forward primer and GFP reverse primer by using the above PCR product 1 and PCR product 2 as templates.

GR1 and GFP were linked together by G4S linker. GR1 and GFP were fused together and the resultant amplified fragments were digested with the restriction enzymes Nde1 and EcoR1Nde1 an EcoR1 (950 bp). And the fragment was inserted in the vector digested with the same enzymes as the above. DNA sequence of the plasmid was confirmed by dideoxy DNA sequencing.

The completed GR1-GFP plasmid was digested with the restriction enzymes Nde1 and Age1, resulting in the preparation of a vector for this experiment. GR4-GFP plasmid was digested with Nde1 and BspE1. The obtained small fragment was inserted in the vector, resulting in the preparation of GR5-GFP plasmid.

GR1-GFP plasmid was digested with the restriction enzymes Nde1 and Age1 by the same method above, resulting in the preparation of a vector for this experiment. GR9-GFP plasmid was digested with Nde1 and BspE1. The obtained fragment was inserted in the vector, resulting in the preparation of GR10-GFP plasmid. DNA sequence of the plasmid was confirmed by dideoxy DNA sequencing.

The protein was over-expressed by the conventional method reported previously (J.H. Park, et al., Mol Cells 12 (2001) 398-402). The lysate was separated by Ni²⁺-chelating sepharose fast flow chromatography (Amersham Bioscience, Sweden), followed by size-exclusion chromatography using Hiload Superdex-75 pg or Hiload Superdex-200 pg(26/60) (Amersham Bioscience, Sweden).

**[Table 8]**

| |
|---|
| Nucleotide sequence of GFP |
| 5'- ATGGTGAGCAAGGGCGAGGAGCTGTTCACCGGGGTGGTGCCCATCCTGGTCGAGCTGG ACGGCGACGTAAACGGCCACAAGTTCAGCGTGTCCGGCGAGGGCGAGGGCGATGCCAC CTACGGCAAGCTGACCCTGAAGTTCATCTGCACCACCGGCAAGCTGCCCGTGCCCTGG CCCACCCTCGTGACCACCCTGACCTACGGCGTGCAGTGCTTCAGCCGCTACCCCGACC ACATGAAGCAGCACGACTTCTTCAAGTCCGCCATGCCCGAAGGCTACGTCCAGGAGCG CACCATCTTCTTCAAGGACGACGGCAACTACAAGACCCGCGCCGAGGTGAAGTTCGAG GGCGACACCCTGGTGAACCGCATCGAGCTGAAGGGCATCGACTTCAAGGAGGACGGCA ACATCCTGGGGCACAAGCTGGAGTACAACTACAACAGCCACAACGTCTATATCATGGC CGACAAGCAGAAGAACGGCATCAAGGTGAACTTCAAGATCCGCCACAACATCGAGGAC GGCAGCGTGCAGCTCGCCGACCACTACCAGCAGAACACCCCCATCGGCGACGGCCCCG TGCTGCTGCCCGACAACCACTACCTGAGCACCCAGTCCGCCCTGAGCAAAGACCCCAA CGAGAAGCGCGATCACATGGTCCTGCTGGAGTTCGTGACCGCCGCCGGGATCACTCTC GGCATGGACGAGCTGTACAAGTAA-3' (SEQ. ID. NO:: 8) |

**[Table 9]**

| |
|---|
| GR1 Forward primer sequence |
| 5'-GCC CAT ATG CAT CAC CAT CAC-3' (SEQ. ID. NO: 9) |
| GR1 Reverse primer sequence |
| 5'-GCC CTT GCT CAC CAT TCC GGA GGA CCC GCC TCC ACC-3' (SEQ. ID. NO: 10) |

**[Table 10]**

| |
|---|
| GFP Forward primer sequence |
| 5'-GCC TCC GGA ATG GTG AGC AAG GGC GAG-3' (SEQ. ID. NO: 11) |
| GFP Reverse primer sequence |
| 5'-GCC GAA TTC TTA CTT GTA CAG CTC GTC-3' (SEQ. ID. NO: 12) |

### Example 14: Amplification of antibody signal by the repeating chains GR, AR, LA, and LAR confirmed by Western blotting

Western blotting was performed by the same manner as described in Example 3.

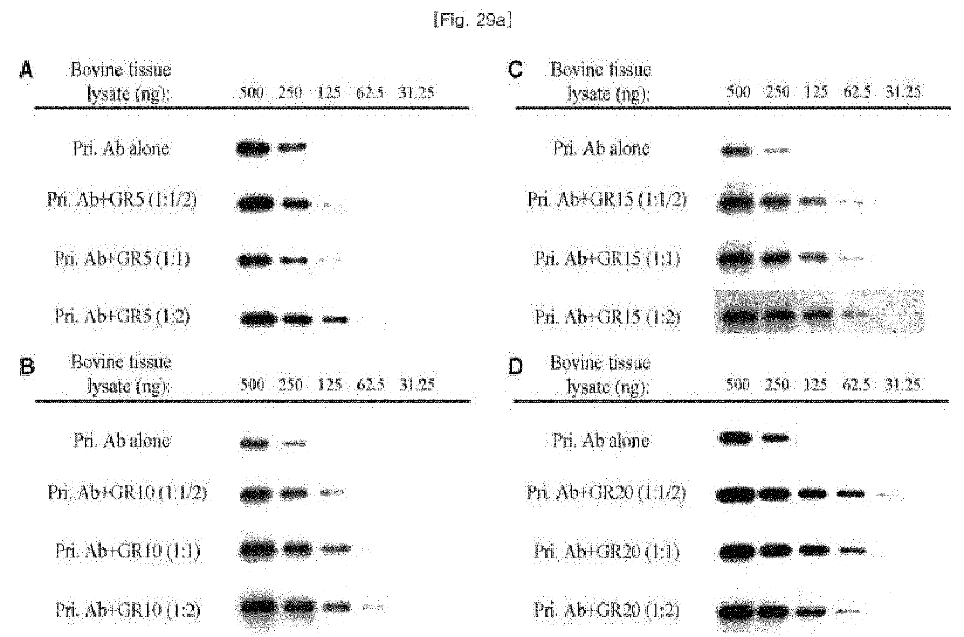

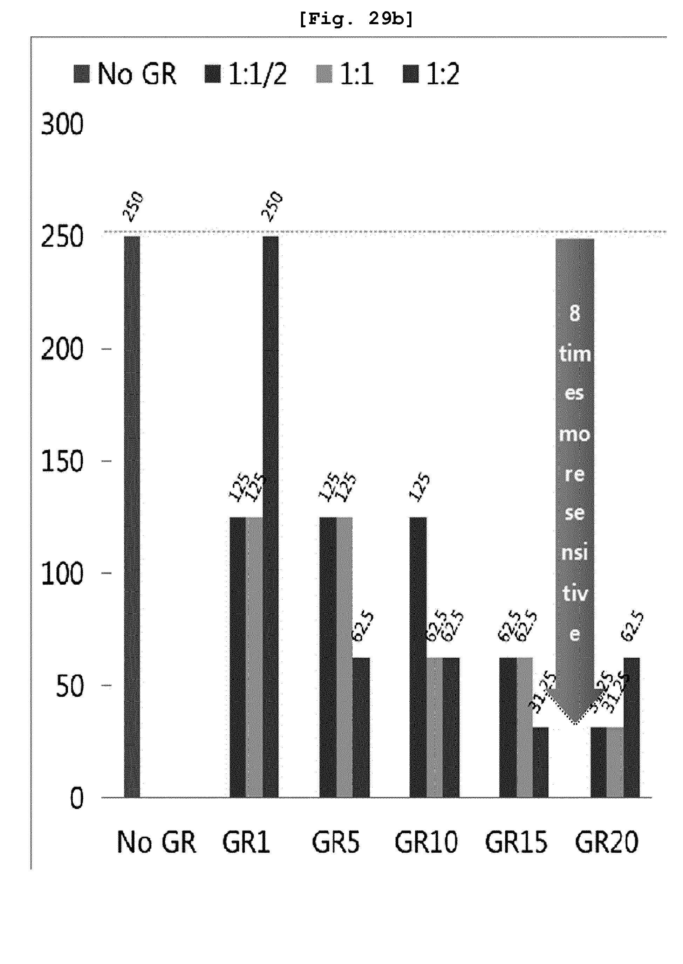

As shown in Figure 29a, the detection limit of Western blotting was extended by GR, confirmed by the comparison with the detection limit of Western blotting without using GR. Herein, GR5, 10, 15, and 20 were used. The molar ratios of them are as shown in the Figure. It was also confirmed that the optimum ratio was changed by the change of the length of repeating chain. As the repeating chain became longer, the amplification effect was increased and thereby the sensitivity was increased. When GR20 was used, the sensitivity was increased 8 times more. In Figure 29b, the content of Figure 29a is schematized displaying the comparison of the detectable minimum antigen concentration (Figure 29b).

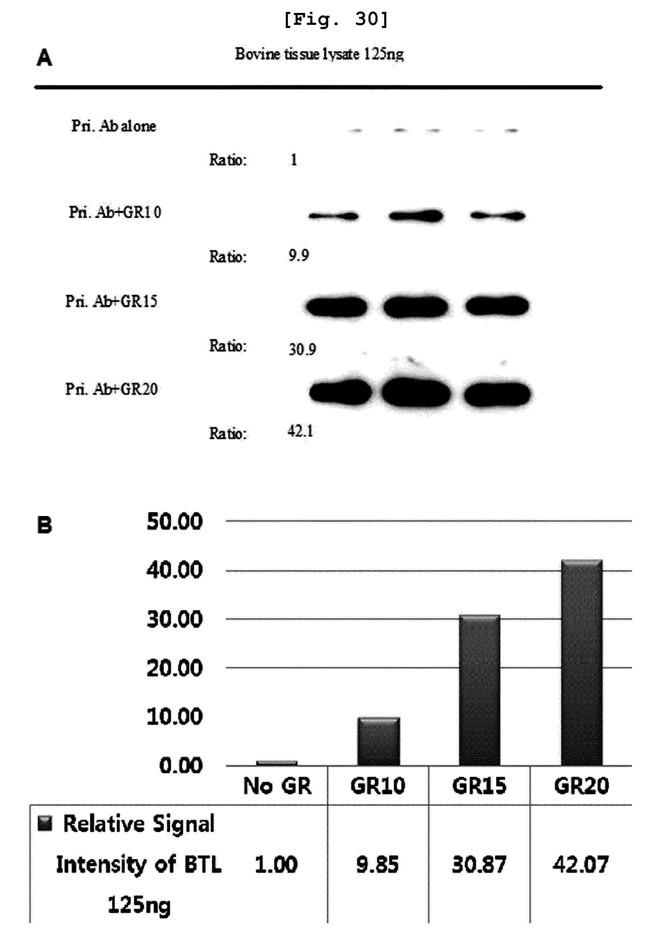

As shown in Figure 30A, the chemiluminescence signal was amplified by GR even in the presence of the same amount of antigen, confirmed by Western blotting. When GR20 was used, it formed a super-complex with the primary Ab, which caused the amplification of the signal as high as at maximum 42 times (Figure 30). Figure 30B is the graph illustrating the content of Figure 30A (Figure 30).

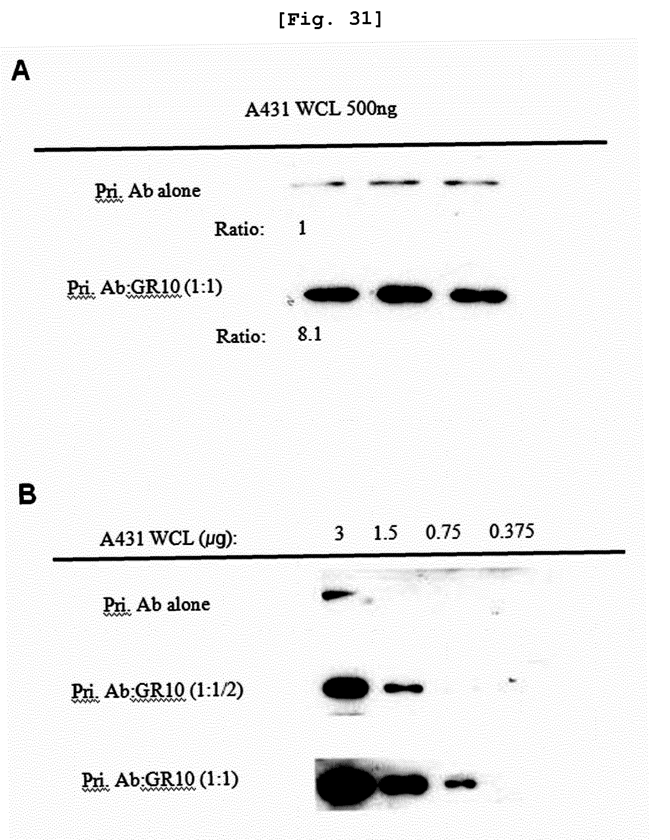

Figure 31 presents the signal amplification by the formation of GR super-complex in Western blotting with different types of antigens. At this time, A431 whole cell lysate was used instead of the conventional bovine tissue lysate and as a result the amplification of signal was also confirmed (Figure 31).

Figure 31B presents that the amplification of Western blot signal could be possible by the repeating chain in the presence of a different primary antibody. At this time, anti-E-cadherin mouse monoclonal antibody was used instead of anti-β-actin mouse monoclonal antibody (Figure 31).

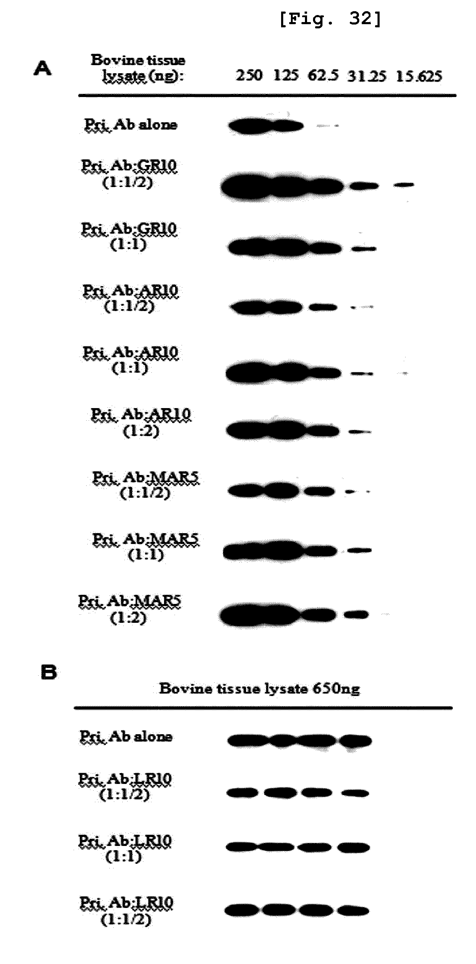

Figure 32A presents the comparison of the amplification levels in Western blotting by different repeating chains. The amplification caused by each GR10, AR10, and MAR5 (repeating chain having a different type of L from LAR5) was compared under the same condition. When AR10 was compared with MAR5, the amplification effect was greater when MAR5 wherein two different domains (A and M) were repeated 5 times was used than when AR10 wherein only one domain (A) was repeated 10 times was used (Figure 32).

Figure 32B presents the result of Western blotting using LR10. Unlike the other repeating chains, LR did not cause any big changes in the amplification (Figure 32).

### Example 15: Investigation of cross-binding between repeating chain and gold antibody complex or super-complex and detection line antibody by using rapid antigen test

The most representative detection method using an antibody, influenza rapid antigen test kit was used to detect gold antibody by using a cross-binding between the repeating chain linked gold antibody complex or the super-complex and the detection line antibody in the absence of an antigen. The detection line generated therein was due to the cross-binding between the detection line antibody and the empty space of the antibody binding domain of the repeating chain of the repeating chain/gold antibody complex or the super-complex. Herein, the influenza antigen test kit provided by SD Bioline and the influenza antigen test kit provided by Korea Green Cross Co were used. The kit included antigen buffer, cotton swab for the collection of sample, and strip. The antigen buffer was opened and the buffer was poured in a tube. A series of GR, AR, LR, and MAR repeating chain was added to the tube containing the antigen buffer, followed by mixing at least 5 times. The strip was added into the tube, which was read 10 ~ 15 minutes later.

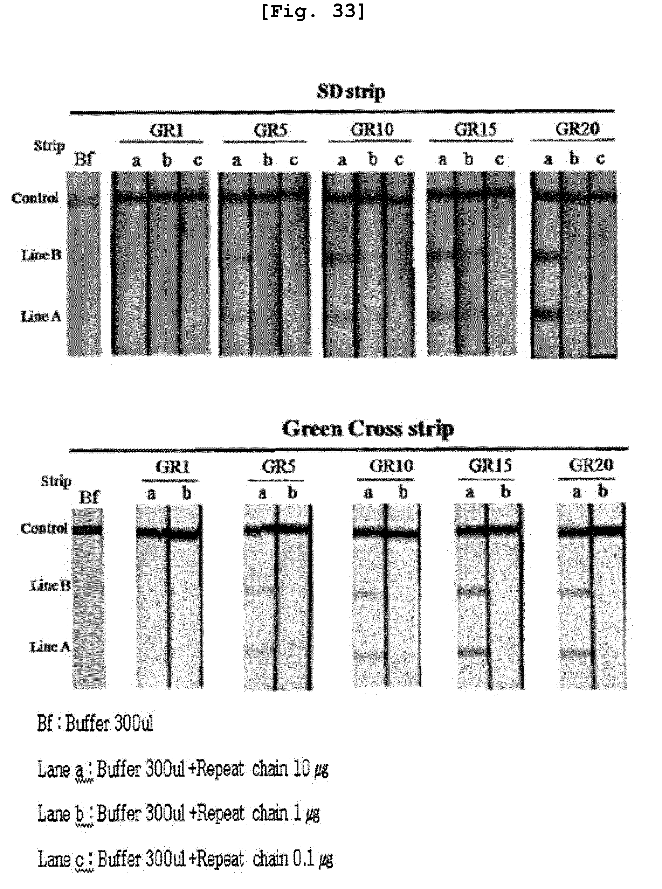

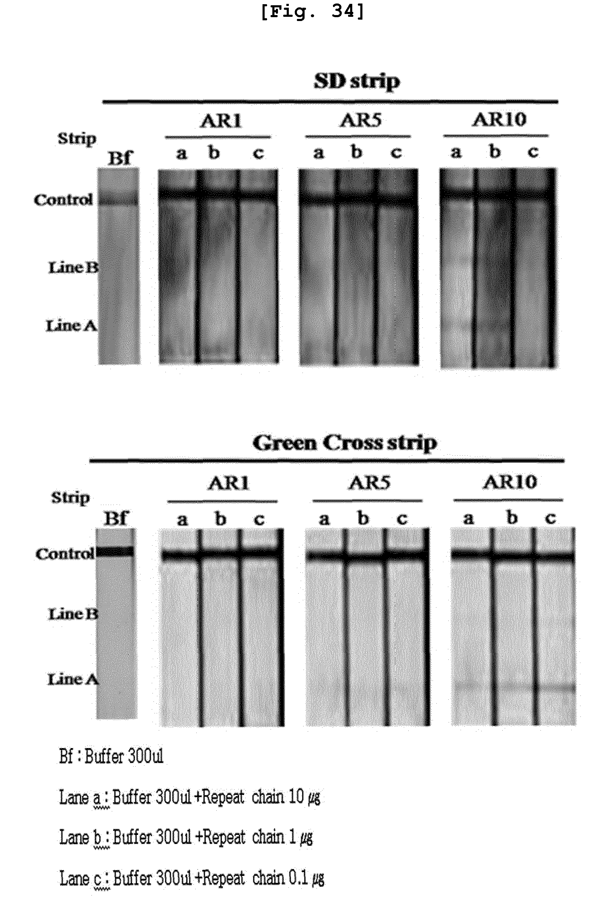

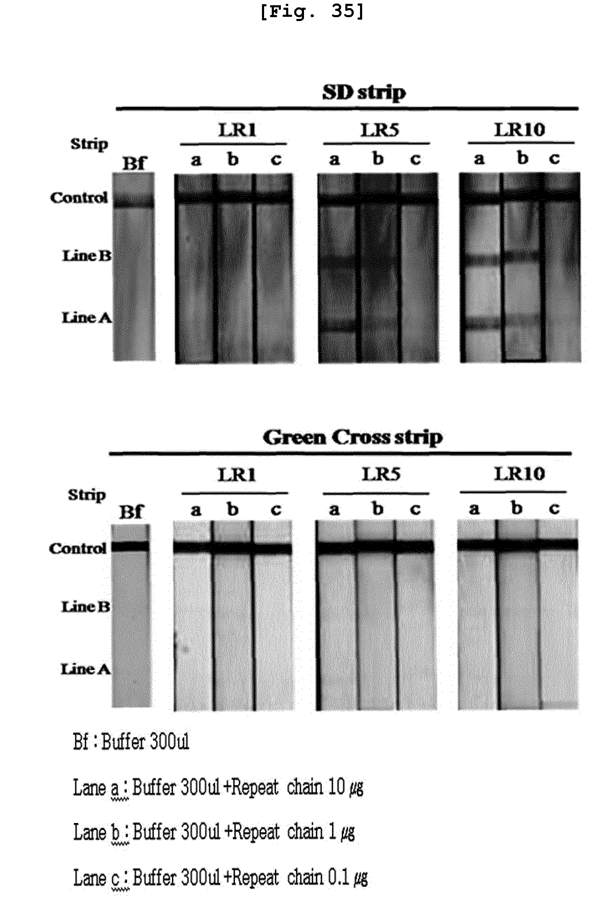

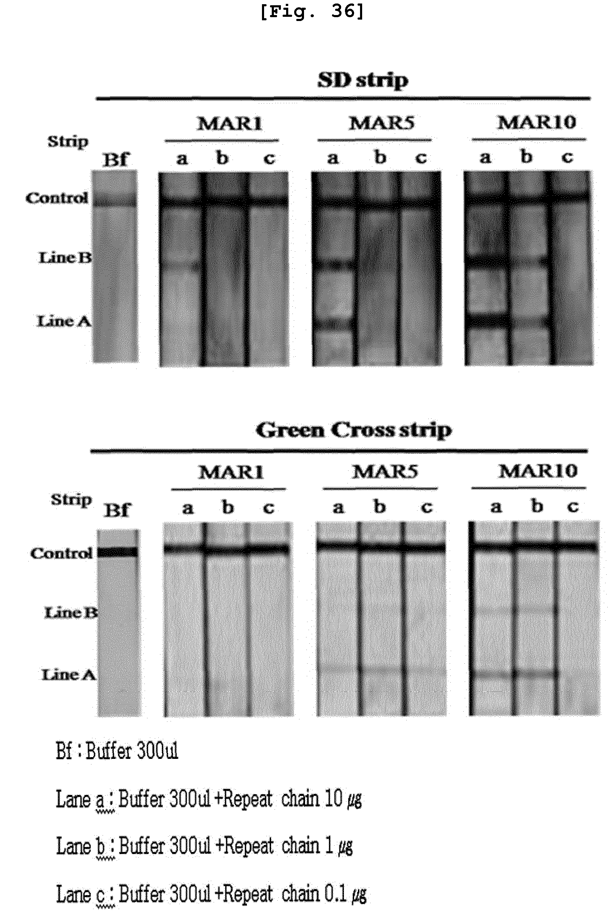

In the case of GR, GR5, GR10, GR15, and GT20 produced a gold line on each strip (Figure 33). In the case of AR, AR10 produced gold lines on all the strips. AR5 did not produce a gold line at any concentration (Figure 34). In the case of LR, LR5 and LR10 produced a gold line on SD strip but no LR protein displayed a cross-binding on Korea Green Cross strip. This was because the antibody VL provided by Korea Green Cross Co was not the kappa chain to which LR protein could bind (Figure 35). In the case of MAR (repeating chain having a different type of L from LAR5), MAR1, MAR5, and MAR10 produced a gold line on SD strip, and MAR5 and MAR10 produced a gold line on Korea Green Cross strip. There was a difference in the gold line formation pattern between the above two kinds of strips. A much stronger gold line was observed on SD strip than on Korea Green Cross strip (Figure 36). The pattern was changed according to whether or not the detection line antibody was VL kappa chain to which L binding domain could bind. On Korea Green Cross strip wherein the detection line antibody did not contain VL kappa, MAR10 gold line was weaker than that shown on SD strip because the MAR10 gold line was resulted only from A binding domain prepared from protein A domain B.

GR1, AR1, and LA1, all of which included one domain each, did not form a gold line on any of those strips because the cross-binding between gold antibody and detection line antibody was not possible with only one domain.

### Example 16: Amplification of antibody signal by GRN (1, 5, 10, 15, 20), ARN (1, 5, 10), LRN (1, 5, 10), and MARN (1, 5, 10) confirmed by enzyme-linked immunosorbent assay (ELISA)

A bovine tissue lysate was diluted in coating buffer at the final concentration of 100 ng/mℓ. The diluted lysate was loaded in a microtiter plate (50 µℓ/well) by pipetting, leading to the coating of the plate. The plate was covered with a lid and kept warm at 4 °C for overnight. Then, the coating buffer was eliminated and the plate was washed twice with 200 µℓ of washing buffer (phosphate buffered saline). The washing buffer (PBS containing 0.1% (v/v) Tween 20) was eliminated by shaking the plate in the sink. The remaining water drop was eliminated by paper towel with tapping softly. For blocking the plate, 200 µℓ of blocking solution (3% bovine serum albumin (BSA), phosphate buffered saline) was added to each well of the plate to block the protein binding site remaining on the coated well. After covering the plate with a lid, the plate stayed warm at room temperature for 2 hours, followed by washing with PBS twice. 100 µℓ of the mixture of dilution buffer/primary antibody (mouse anti-actin antibody (Santacruz Biotech)) or the mixture of primary antibody/repeating chain was added thereto right before use. After covering the plate, the plate stayed warm again at room temperature for 1 hour, followed by washing with PBS twice. 100 µℓ of the mixture of dilution buffer/Goat anti-mouse beta actin-HRP antibody (1:2000) was added right before use. The plate was covered again and kept warm at room temperature for 1 hour, followed by washing with PBS twice. 100 µℓ of TMB (HRP substrate) was added thereto, and the plate stayed warm at room temperature for 20 minutes. 100 *µℓ* of 2 M H₂SO₄ was added thereto, and then plate reading was performed at 450 nm with a plate reader.

It was confirmed that the signal amplification was induced by GR5, GR10, GR15, and GR20. In the case of using GR5, when it was mixed with the primary antibody at the molar ratio of 1:2, the signal amplification effect was the greatest, suggesting that the most effective complex was formed. The amplification was 6 times higher than the regular antibody signal. In the case of using GR10, when it was mixed with the primary antibody at the molar ratio of 1:2, like GR5, the amplification was 9.8 times higher and the effective complexes were also formed by GR15 and GR20 added at the molar ratio of 1:1/2 respectively. At this time, the amplification was respectively 10.5 and 12.9 times higher (Table 11). In the case of using AR and MAR, when AR10 and MAR10 were added at the molar ratio of 1:2 and 1:1, the amplification level was increased 1.8 times and 1.6 times respectively. On the other hand, the amplification effect was not observed in the case of using LR. This was because the primary antibody VL used in this invention was not kappa chain, so that the amplification was not induced.

GR1, AR1, and LR1 which included only one domain, did not cause signal amplification at any concentration. This was because the super-complex could not be formed by a cross-binding between the complexes having only one domain.

**[Table 11]**

| Measurement of antibody signal amplification by GRN (1, 5, 10, 15, 20), ARN (1, 5, 10), LRN (1, 5, 10), and MARN (1, 5, 10) with indirect ELISA | | |
|---|---|---|
| Sample | A450 Value | Fold increase |
| IgG only | 0.188 | 1.0 |
| IgG+GR1 | 0.207 | 1.1 |
| IgG+GR5 | 1.128 | 6.0 |
| IgG+GR10 | 1.842 | 9.8 |
| IgG+GR15 | 1.974 | 10.5 |
| IgG+GR20 | 2.425 | 12.9 |
| IgG+AR1 | 0.195 | 1.0 |
| IgG+AR5 | 0.183 | 1.0 |
| IgG+AR10 | 0.338 | 1.8 |
| IgG+LR1 | 0.190 | 1.0 |
| IgG+LR5 | 0.189 | 1.0 |
| IgG+LR10 | 0.188 | 1.0 |
| IgG+MAR1 | 0.191 | 1.0 |
| IgG+MAR5 | 0.190 | 1.0 |
| IgG+MAR10 | 0.300 | 1.6 |

### Example 17: Preparation of [B3(Fab)-ext-PE38]₂ from the complex of protein G domain III repeating chain recombinant protein and B3(Fab)-ext-PE38

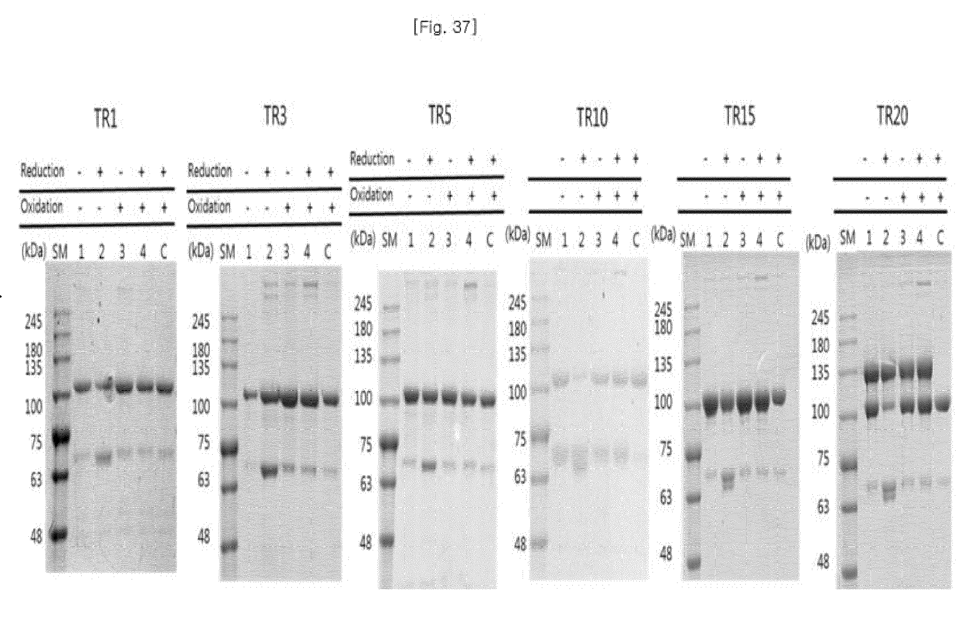

To construct [B3(Fab)-ext-PE38]₂ by using the complex of the purified TR1, 3, 5, 10, 15, and 20 (= GR1, 3, 5, 10, 15, 20) proteins and B3(Fab)-ext-PE38, the complex was reduced by 2-mercaptoethanol and then oxidized into glutathione oxidized form (GSSG), followed by analysis with non-reducing SDS-PAGE (Figure 37).

Particularly, the protein complex was fixed on metal chelating sepharose beads at 10 °C for 1 hour. 40 µℓ of the metal chelating sepharose beads [suspended in 100 mM Tris-HCl (pH 8.2) at the concentration of 50%] was added to each reaction mixture. The fixed protein complex was added with 100 mM Tris-HCl (pH 8.2) supplemented with 40 mM 2-merchaptoethanol at room temperature to induce reduction. To determine the concentration of 2-mercaptoethanol necessary for the reduction of cysteine residue of B3(Fab)-ext-PE38, a preliminary experiment was first performed, wherein the complete reduction of B3(Fab)-ext-PE38 cysteine residue was confirmed in the presence of 2-mercaptoethanol at the concentration of 20 ~ 40 mM. The reduced protein complex was washed with washing buffer containing 100 mM MOPS (pH 6.5) once, and then washed three times more with 100 mM Tris-HCl (pH 8.2). After washing, the protein complex was oxidized with the oxidizing buffer comprising 5 mM GSSG and 100 mM Tris-HCl (pH 8.2). Upon completion of the oxidization, the complex stayed warm at 37°C for 2 hours. Thereafter, 2X SDS sample buffer was added thereto, followed by SDS-PAGE and Coomassie staining. The density was analyzed by using a densitometer to investigate the yield of [B3(Fab)-ext-PE38]₂. The increase rate of [B3(Fab)-ext-PE38]₂ was calculated by dividing the value of strength of the band of [B3(Fab)-ext-PE38]₂ finished with the oxidization/reduction on SDS-PAGE in the presence of TR (Figure 37, Lane 4) by the value of strength of the band of [B3(Fab)-ext-PE38]₂ finished with the oxidization/reduction in the absence of TR (Figure 37, Lane 5) (Table 12).

As a result, the band strength of [B3(Fab)-ext-PE38]₂ was not increased when it was alone or in the form of the complex with TR1. In the complexes with TR3, 5, 10, 15, and 20, the increased collision frequency between monomers induced the production of disulfide-bridged dimer. The production of [B3(Fab)-ext-PE38]₂ in the complexes with TR3, TR5, TR10, TR15, and TR20 was 4.6 times, 3.4 times, 16.7 times, 3.5 times, and 2.7 times increased. The production of [B3(Fab)-ext-PE38]₂ was achieved within 2 hours from the addition of an oxidizing agent. However, the additional time for warming up did not increase a significant increase of the production. Therefore, it was confirmed that the cysteine residue in the molecule [B3(Fab)-ext-PE38] binding to TR protein could approach and bind easily to the protein through the repeating chain.

**[Table 12]**

| Increase rate of [B3(Fab)-ext-PE38]₂ production | |
|---|---|
| Antibody-toxin | Increase rate of production (times) |
| B3(Fab)-ext-PE38 | Not detected |
| B3(Fab)-ext-PE38: TR1 | Not detected |
| B3(Fab)-ext-PE38: TR3 | 4.6 |
| B3(Fab)-ext-PE38: TR5 | 3.6 |
| B3(Fab)-ext-PE38: TR10 | 16.7 |
| B3(Fab)-ext-PE38: TR15 | 3.5 |
| B3(Fab)-ext-PE38: TR20 | 2.7 |

### Example 18: Preparation of [Herceptin(Fab)-ext-PE38]₂ from the complex of protein G domain III repeating chain recombinant protein and Herceptin(Fab)-ext-PE38

To construct [Herceptin(Fab)-ext-PE38]₂ by using the complex of the purified GR5, GR10, GR15, and GR20 (= TR5, TR10, TR15, and TR20) proteins and Herceptin(Fab)-ext-PE38, the complex was reduced by 2-mercaptoethanol and then oxidized into glutathione oxidized form (GSSG), followed by analysis with SDS-PAGE.

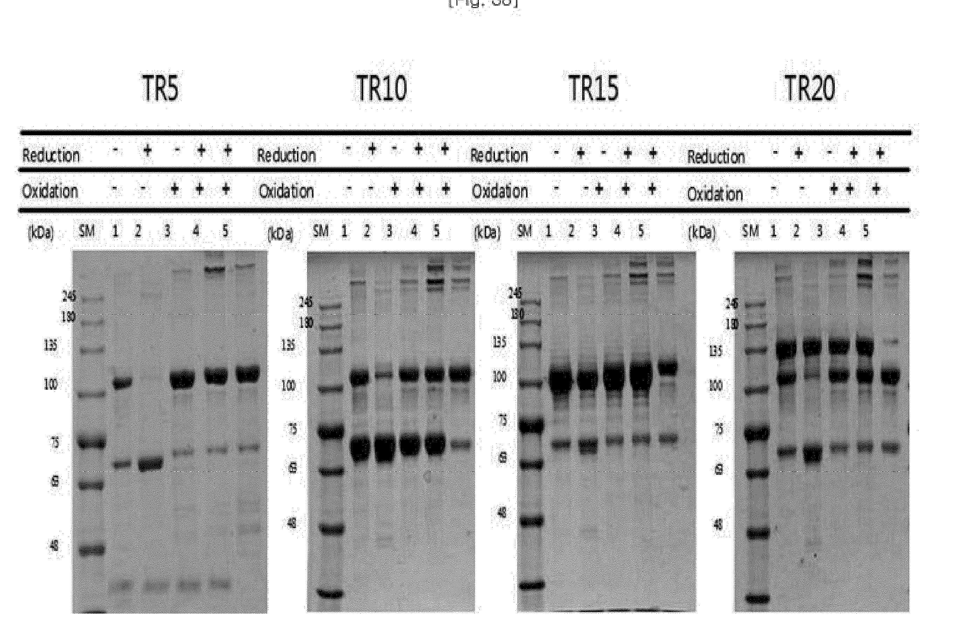

Particularly, the protein complex was fixed on metal chelating sepharose beads at 4 °C for 1 hour (Figure 38, lane 1). 40 µℓ of the metal chelating sepharose beads [suspended in 100 mM Tris-HCl (pH 8.2) at the concentration of 50%] was added to each reaction mixture. The fixed protein complex was added with 100 mM Tris-HCl (pH 8.2) supplemented with 40 mM 2-merchaptoethanol at room temperature to induce reduction (Figure 38, lane 2). The reduced protein complex was washed with washing buffer containing 100 mM MOPS (pH 6.5) once, and then washed three times more with 100 mM Tris-HCl (pH 8.2). After washing, the protein complex was oxidized with the oxidizing buffer comprising 5 mM GSSG and 100 mM Tris-HCl (pH 8.2). Upon completion of the oxidization, the complex stayed warm at 37°C for 2 hours (Figure 38, lane 3). Thereafter, 2X SDS sample buffer was added thereto, followed by SDS-PAGE and Coomassie staining. The density was analyzed by using a densitometer to investigate the yield of [Herceptin(Fab)-ext-PE38]₂ (Table 13). The increase rate of [Herceptin(Fab)-ext-PE38]₂ was calculated by dividing the value of strength of the band of [Herceptin(Fab)-ext-PE38]₂ finished with the oxidization/reduction on SDS-PAGE in the presence of TR (Lane 4) by the value of strength of the band of [Herceptin(Fab)-ext-PE38]₂ finished with the oxidization/reduction in the absence of TR (Lane 5) (Table 13). At this time, Herceptin(Fab)-ext-PE38 that had been reduced only (Figure 38, Lane 2), Herceptin(Fab)-ext-PE38 that had been oxidized only (Figure 38, Lane 3) and Herceptin(Fab)-ext-PE38 alone (-TR) were used as the negative controls.

As a result, the production of [Herceptin(Fab)-ext-PE38]₂ was 2.3 times, 2.3 times, 1.7 times, and 3.3 times higher in the complexes with TR5, TR10, TR15, and TR20 than in Herceptin(Fab)-ext-PE38 alone (-TR). In the complexes with TR5, TR10, TR15, and TR20, the increased collision frequency between monomers induced the production of disulfide-bridged dimer. The production of [Herceptin(Fab)-ext-PE38]₂ was achieved within 2 hours from the addition of an oxidizing agent. However, the additional time for warming up did not increase a significant increase of the production. Therefore, it was confirmed that the cysteine residue in the molecule [B3(Fab)-ext-PE38] binding to TR protein could approach and bind easily to the protein through the repeating chain.

**[Table 13]**

| Increase rate of [Herceptin(Fab)-ext-PE38]₂ | |
|---|---|
| Antibody-toxin | Increase rate |
| Herceptin(Fab)-ext-PE38 + no TR | 1.0 |
| Herceptin (Fab)-ext-PE38 + TR5 | 2.3 |
| Herceptin (Fab)-ext-PE38 + TR10 | 2.3 |
| Herceptin (Fab)-ext-PE38 + TR15 | 1.7 |
| Herceptin (Fab)-ext-PE38 + TR20 | 3.3 |

### Example 19: Cytotoxic effect of the complex of GR repeating chain protein and [Herceptin(Fab)-ext-PE38] monomer

The over-expression, preparation, and refolding of Herceptin(Fd)-ext-PE38, H6-Herceptin(L), e23(Fd)-ext-PE38, and H6-e23(L) inclusion body were performed by the conventional methods reported earlier (J.H. Park, et al., Mol Cells 12 (2001) 398-402). To confirm the cytotoxic effect after the association with GR protein, [Herceptin(Fab)-ext-PE38] was associated with GR5 ~ GR20.

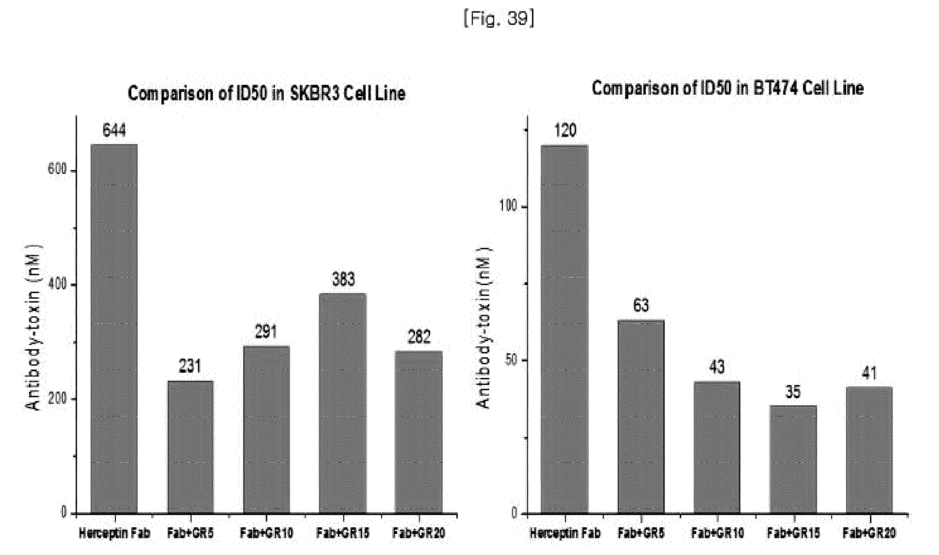

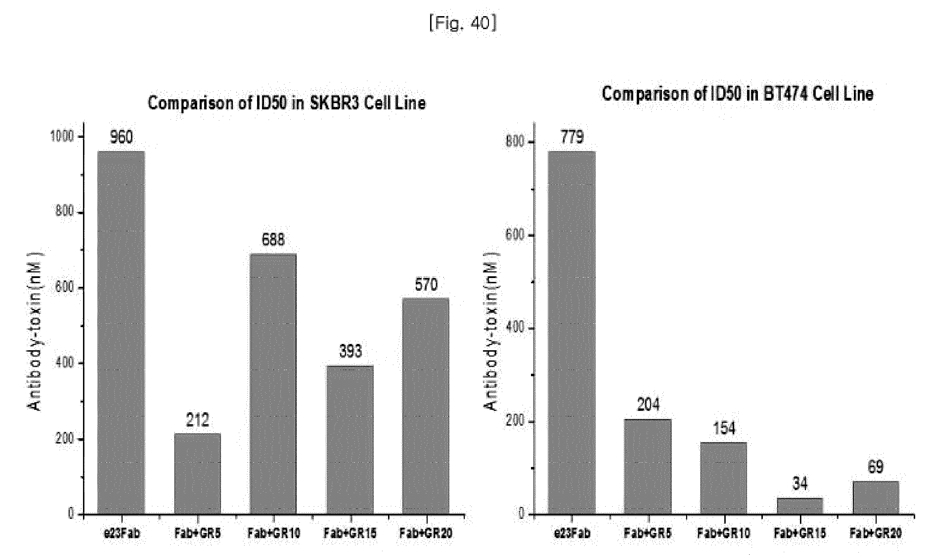

Particularly, [Herceptin(Fab)-ext-PE38] and TR5 ~ TR20, and [e23(Fab)-ext-PE38] and TR5 ~ TR20 were cultured at 37°C for 1 hour. They were treated to breast cancer cells at different concentrations, followed by reaction at 37 °C for 24 hours. Then, CCK-8 solution was treated to the medium (1/10 of the medium), followed by reaction at 37°C for 4 hours. Then, the effect was observed (Figures 39 and 40, Tables 14 and 15).

The cytotoxic effect of the complexes with TR5, TR10, TR15, and TR20 on SKBR3 cells was respectively 2.8 times, 2.2 times, 1.7 times, and 2.3 times higher than that of Herceptin(Fab)-ext-PE38 alone. The cytotoxic effect of the complexes with TR5, TR10, TR15, and TR20 on SKBR3 cells was respectively 4.5 times, 1.4 times, 2.4 times, and 1.7 times higher than that of e23(Fab)-ext-PE38 alone. The cytotoxic effect of the complexes with TR5, TR10, TR15, and TR20 on BT474 cells was respectively 1.9 times, 2.8 times, 3.4 times, and 2.9 times higher than that of Herceptin(Fab)-ext-PE38 alone. And the cytotoxic effect of the complexes with TR5, TR10, TR15, and TR20 on SKBR3 cells was respectively 3.8 times, 5 times, 23 times, and 11.3 times higher than that of e23(Fab)-ext-PE38 alone.

**[Table 14]**

| SKBR3 cell line | | BT 474 cell line | |
|---|---|---|---|
| Herceptin(Fab)-PE38 + no GR | 1.0 | Herceptin(Fab)-PE38 + no GR | 1.0 |
| Herceptin(Fab)-PE38 + GR5 | 2.8 | Herceptin(Fab)-PE38 + GR5 | 1.9 |
| Herceptin(Fab)-PE38 + GR10 | 2.2 | Herceptin(Fab)-PE38 + GR10 | 2.8 |
| Herceptin(Fab)-PE38 + GR15 | 1.7 | Herceptin(Fab)-PE38 + GR15 | 3.4 |
| Herceptin(Fab)-PE38 + GR20 | 2.3 | Herceptin(Fab)-PE38 + GR20 | 2.9 |

**[Table 15]**

| SKBR3 cell line | | BT 474 cell line | |
|---|---|---|---|
| e23(Fab)-PE38 + no GR | 1.0 | e23(Fab)-PE38 + no GR | 1.0 |
| e23(Fab)-PE38 + GR5 | 4.5 | e23(Fab)-PE38 + GR5 | 3.8 |
| e23(Fab)-PE38 + GR10 | 1.4 | e23(Fab)-PE38 + GR10 | 5.0 |
| e23(Fab)-PE38 + GR15 | 2.4 | e23(Fab)-PE38 + GR15 | 23.0 |
| e23(Fab)-PE38 + GR20 | 1.7 | e23(Fab)-PE38 + GR20 | 11.3 |

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended Claims.

## Claims

1. A method for preparing a repeating chain for the production of a super-complex containing the step of producing a repeating chain which comprises a single type of binding domains or multiple types of binding domains having at least two binding sites in a monomer and a binding specificity to the monomer.

2. A method for preparing a complex of multiple numbers of monomers/repeating chain for the production of a super-complex, which comprises the following steps:
1) preparing a repeating chain wherein a single type of binding domains or multiple types of binding domains having at least two binding sites in a monomer and a binding specificity to the monomer are repeated; and
2) preparing a complex of multiple numbers of monomers/repeating chain by mixing the repeating chain of step 1) and the monomer having at least two binding sites binding to the said repeating chain.

3. A method for preparing a super-complex, which comprises the following steps:
1) preparing a repeating chain wherein a single type of binding domains or multiple types of binding domains having at least two binding sites in a monomer and a binding specificity to the monomer are repeated;
2) preparing a complex of multiple numbers of monomers/repeating chain by mixing the repeating chain of step 1) and the monomer having at least two binding sites binding to the said repeating chain; and
3) forming an aggregate of the said complexes by forming a cross-binding between the complexes of multiple numbers of monomers/repeating chain of step 2).

4. The method according to one of claim 1 ~ claim 3, wherein the monomer is a protein.

5. The method according to one of claim 1 ~ claim 3, wherein the monomer is selected from the group consisting of antibodies, ligands, receptors, fragments thereof, recombinants thereof, derivatives thereof, and biological/chemical effector group conjugates.

6. The method according to claim 5, wherein the antibody is selected from the group consisting of antibody fragments, Fab fragments, Fab fragment containing fragments, Fv fragments, Fv fragment containing fragments, Fc fragments, and Fc fragment containing fragments.

7. The method according to one of claim 1 ~ claim 3, wherein the binding domain is a protein.

8. The method according to one of claim 1 ~ claim 3, wherein the binding domain is derived from a microorganism protein.

9. The method according to one of claim 1 ~ claim 3, wherein the binding domain is selected from the group consisting of streptococcal protein G, Staphylococcus aureus protein A, Peptostreptococcus magnus protein L, and derivatives thereof.

10. A repeating chain prepared by the method of claim 1.

11. A complex of multiple numbers of monomers/repeating chain prepared by the method of claim 2.

12. A super-complex prepared by the method of claim 3.

13. A method for amplifying the effect of a monomer, containing the step of forming a super-complex binding to the monomer target by mixing the repeating chain of claim 10, the complx of multiple monomers/repeating chain of claim 11 or the super-complex of claim 12.

14. The method for amplifying the effect of a monomer according to claim 13, wherein the method includes an additional step of measuring the effect of a monomer on the monomer target.

15. The method for amplifying the effect of a monomer according to claim 13 or claim 14, wherein the target of the monomer is selected from the group consisting of antigens, antibodies, peptides, proteins, bacteria, viruses, and fungi.

16. The method for amplifying the effect of a monomer according to claim 15, wherein the bacteria are selected from the group consisting of Helicobacter pylori, Mycobacterium tuberculosis, and Chlamydia trachomatis.

17. The method for amplifying the effect of a monomer according to claim 15, wherein the virus is selected from the group consisting of influenza, foot-and-mouth disease virus, human papilloma virus (HPV), dengue fever virus, hepatitis C virus, hepatitis B surface antigen, and hepatitis B surface antibody.

18. The method for amplifying the effect of a monomer according to claim 14, wherein the effect of a monomer is measured by using the biological and chemical labeling function of the secondary probe(antibody)-marker conjugate and the marker.

19. The method for amplifying the effect of a monomer according to claim 18, wherein the marker is selected from the group consisting of horseradish peroxidase (HRP), alkaline phosphatase, colloid gold, fluorescein, Quantum dot, glucose oxidase, luciferase, beta-D-galactosidase, malate dehydrogenase (MDH), acetylcholinesterase, isotope, and dye.

20. The method for amplifying the effect of a monomer according to claim 18, wherein the substrate is selected from the group consisting of 3,3', 5,5'-tetramethyl bezidine (TMB), 2,2'-azino-bis(3-ethylbenzothiazoline-6-sulfonic acid) (ABTS), o-phenylenediamine (OPD), diaminobenzidine (DAB), 3-amino-9-ethylcarbasole, 5-bromo-4-chloro-3-indolyl phosphate/iodonitrotetrazolium (BCIP/INT), new fuchin (NF), and fast red TR salts

21. An analysis kit that is a target specific and contains multiple numbers of monomers wherein each monomer has at least two binding sites and contains a repeating chain of a binding domain that binds specifically to the monomer.

22. The analysis kit according to claim 21, wherein the kit is composed of the followings:
1) repeating chain of binding domain having a binding specificity to monomer;
2) monomer specifically binding to the detection target;
3) secondary probe conjugate labeled with a marker activated by the reaction with a substrate;
4) substrate solution to react with the marker;
5) washing buffer for each reaction stage; and
6) stop solution to terminate the enzyme reaction.

23. The analysis kit according to claim 21 or claim 22, wherein the kit is used for the analysis method selected from the group consisting of immunohistochemical techniques, immunoblot, immunoprecipitation, enzyme linked immunosorbent assay (ELISA), agglutination, immunochromatographic assay, and radio-immuno assay.

24. A method for preparing a repeating chain-detection functional group containing the step of linking, conjugating, or fusing a detection functional group to a repeating chain of a binding domain that binds to a monomer.

25. A method for preparing a complex of multiple monomers/repeating chain-detection functional group comprising the following steps:
1) preparing a repeating chain-detection functional group by linking, conjugating, or fusing a detection functional group to a repeating chain of a binding domain that binds to a monomer; and
2) preparing a complex of multiple monomers/repeating chain-detection functional group by mixing multiple monomers with the repeating chain-detection functional group prepared in step 1).

26. A method for preparing a super-complex, which comprises the following steps:
1) preparing a repeating chain-detection functional group by linking, conjugating, or fusing a detection functional group to a repeating chain of a binding domain that binds to a monomer;
2) preparing a complex of multiple monomers/repeating chain-detection functional group by mixing a monomer having at least two binding sites for the repeating chain with the repeating chain-detection functional group prepared in step 1); and
3) forming an aggregate of the said complexes by forming a cross-binding between the complexes of multiple monomers/repeating chain-detection functional group of step 2).

27. The method according to claim 24, claim 25, or claim 26, wherein the detection functional group is selected from the group consisting of Cy-3, Cy-5, FITC, GFP (green fluorescent protein), RFP (red fluorescent protein), and Texas Red.

28. A repeating chain-detection functional group of a monomer binding domain prepared by the method of claim 24.

29. A complex of multiple monomers/repeating chain-detection functional group prepared by the method of claim 25.

30. A super-complex prepared by the method of claim 26.

31. A method for detecting a target of a monomer containing the step of forming a super-complex by mixing the complex of multiple monomers/repeating chain-detection functional group of claim 29 or the super-complex of multiple monomers/repeating chain-detection functional group of claim 30 with a target antigen.

32. The method for detecting a target of a monomer according to claim 31, wherein the method includes an additional step of measuring the target detection level of a monomer.


SEQUENCE LIST

<110>	CHOE, MuHyeon

<120>	super-complex formed by the cross-binding between the complexes
	of a repeat-chain and multiple monomers, and use thereof

<130>	14fpo-05-14/PCT

<150>	KR 10-2013-0061869
<151>	2013-05-30

<160>	12

<170>	KopatentIn 2.0

<210>	1
<211>	43
<212>	DNA
<213>	Artificial Sequence

<220>
<223>	P3 Primer

<400>	1
agacctttac ggtaactcaa accggtggag gcgggtccgg ata	43

<210>	2
<211>	43
<212>	DNA
<213>	Artificial Sequence

<220>
<223>	P4 Primer

<400>	2
tatccggacc cgcctccacc ggtttcagtt accgtaaagg tct	43

<210> 3
<211> 261
<212> DNA
<213> Artificial Sequence

<220>
<223> Nucleic acid sequence of Protein A Domain B

<400> 3
catatgcatc atcatcatca ccacaccggt tctcaagccc caaaagccga caataaattt 60

aataaagagc agcagaacgc gttttatgaa atcttgcatc tgccgaatct gaatgaagaa 120

caacgtaacg gattcattca gagccttaaa gatgatccta gtcagtccgc taacttactc 180

gcagaagcta agaaactgaa tgatgcacag gcgccgaagg gagggggtgg atccggtggt 240

ggcggctccg gataagaatt c 261

<210> 4
<211> 5
<212> PRT
<213> Artificial Sequence

<220>
<223> G4S

<400> 4
Gly Gly Gly Gly Ser
1               5

<210> 5
<211> 315
<212> DNA
<213> Artificial Sequence

<220>
<223> Nucleic acid sequence of Protein L Domain B1

<400>	5
catatgcatc accatcacca tcataccggt atcaagttcg ccggtaaaga agaaacgccg	60

gaaacccctg agacagacag tgaagaggaa gtgacaataa aagcaaatct gattttcgcc	120

aacgggtcaa cccagacggc cgaattcaaa gggacatttg aaaaagcaac ttctgaggct	180

tatgcatacg cggacactct gaagaaggat aatggtgaat ataccgtaga tgttgctgat	240

aaaggttata ccctgaatat taaatttgcg ggtggcggcg gcggaagcgg tggcggaggt	300

tccggataag aattc	315

<210>	6
<211>	537
<212>	DNA
<213>	Artificial Sequence

<220>
<223>	Nucleic acid sequence of Domain B1-Domain B of protein LA

<400>	6
catatgcatc accatcacca tcataccggt atcaagttcg ccggtaaaga agaaacgccg	60

gaaacccctg agacagacag tgaagaggaa gtgacaataa aagcaaatct gattttcgcc	120

aacgggtcaa cccagacggc cgaattcaaa gggacatttg aaaaagcaac ttctgaggct	180

tatgcatacg cggacactct gaagaaggat aatggtgaat ataccgtaga tgttgctgat	240

aaaggttata ccctgaatat taaatttgcg ggtggcggcg gcggaagcgg tggcggaggt	300

tccggttctc aagccccaaa agccgacaat aaatttaata aagagcagca gaacgcgttt	360

tatgaaatct tgcatctgcc gaatctgaat gaagaacaac gtaacggatt cattcagagc	420

cttaaagatg atcctagtca gtccgctaac ttactcgcag aagctaagaa actgaatgat	480

gcacaggcgc cgaagggagg gggtggatcc ggtggtggcg gctccggata agaattc 537

<210> 7
<211> 4
<212> PRT
<213> Artificial Sequence

<220>
<223> G4

<400> 7
Gly Gly Gly Gly
1

<210> 8
<211> 720
<212> DNA
<213> Artificial Sequence

<220>
<223> Nucleic acid sequence of Fluorescence GFP

<400> 8
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccctgaccta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagtaa 720

720

<210> 9
<211> 21
<212> DNA
<213> Artificial Sequence

<220>
<223> Forward primer sequence of GR1

<400> 9
gcccatatgc atcaccatca c 21

<210> 10
<211> 36
<212> DNA
<213> Artificial Sequence

<220>
<223> Reverse primer sequence of GR1

<400> 10
gcccttgctc accattccgg aggacccgcc tccacc 36

<210> 11
<211> 27
<212> DNA

<213>	Artificial Sequence

<220>
<223>	Forward primer sequence of GFP

<400>	11
gcctccggaa tggtgagcaa gggcgag	27

<210>	12
<211>	27
<212>	DNA
<213>	Artificial Sequence

<220>
<223>	Reverse primer sequence of GFP

<400>	12
gccgaattct tacttgtaca gctcgtc	27